# EUROPÄISCHE PATENTSCHRIFT

(11) **EP 0 280 224 B1**
(45) Veröffentlichungstag und Bekanntmachung des Hinweises auf die Patenterteilung: **01.06.1994**
(21) Anmeldenummer: 88102533.2
(22) Anmeldetag: 22.02.1988
(51) Int. Cl.: C07D 401/14, C07D 403/04, C07D 409/14, C07D 417/04, C07D 413/04, A61K 31/395, C07C 251/72

(54) **Substituierte 3H-Indole, Verfahren zu ihrer Herstellung sowie Arzneimittel**
Substituted 3H-indoles, process for their preparation and medicines containing them
3H-indoles substitués, leur procédé de préparation et médicaments les contenant

(30) Priorität: 27.02.1987 DE 3706427
(43) Veröffentlichungstag der Anmeldung: 31.08.1988
(73) Patentinhaber: BOEHRINGER MANNHEIM GMBH, 68298 Mannheim (DE)
(72) Erfinder: Mertens, Alfred, Dr. rer. nat., D-6905 Schriesheim (DE); Kling, Lothar, Dr., D-6800 Mannheim 31 (DE); Müller-Beckmann, Bernd, Dr. med. vet, D-6718 Grünstadt (DE); Von der Saal, Wolfgang, Dr. rer. nat, D-6940 Weinheim (DE)

(56) Entgegenhaltungen:
- EP-A- 0 122 494
- EP-A- 0 161 918

## Beschreibung

Die vorliegende Erfindung betrifft neue 3H-Indole der allgemeinen Formel I 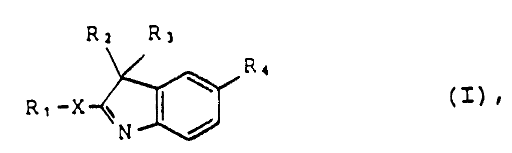
in welcher
R₁ einen Phenylring der allgemeinen Formel II darstellt, 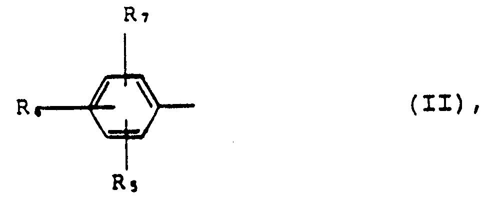
wobei R₅, R₆, R₇ gleich oder verschieden sein können und jeweils Wasserstoff, eine (C₁-C₅)Alkansulfonyloxy-, Trifluormethansulfonyloxy-, (C₁-C₅)Alkansulfonylamino-, Trifluormethansulfonylamino-, N-(C₁-C₅)Alkyl(C₁-C₅)alkansulfonylamino-, N-(C₁-C₅)Alkyltrifluormethansulfonylamino-, (C₁-C₅)Alkylsulfenylmethyl-, (C₁-C₅)Alkylsulfinyl-methyl- oder (C₁-C₅)Alkylsulfonylmethylgruppe, eine durch eine Hydroxy-, (C₁-C₅)Alkoxy-, Amino-, (C₁-C₅)Alkylamino- oder Di(C₁-C₅)alkylaminogruppe substituierte Carbonylgruppe, eine durch eine Amino-, (C₁-C₅)Alkylamino-, Di-(C₁-C₅)alkylamino- oder cyclische Iminogruppe substituierte Sulfonylgruppe, wobei eine Methylengruppe in 4-Stellung durch ein Schwefel- oder Sauerstoffatom ersetzt sein kann, eine (C₁-C₅)Alkylcarbonylamino-, Aminocarbonylamino- oder (C₁-C₅)Alkylaminocarbonylaminogruppe, eine (C₁-C₅)Alkylmercapto-, (C₁-C₅)Alkylsulfinyl- oder (C₁-C₅)Alkylsulfonylgruppe, eine Nitro-, Halogen-, Amino-, Hydroxy-, (C₁-C₅)Alkyl-, (C₁-C₅)Alkoxy-, (C₁-C₅)Alkenyloxy-, (C₁-C₅)Alkinyloxy-, Cyan(C₁-C₅)alkyloxy-, Carboxy(C₁-C₅)-alkyloxy-, (C₁-C₅)Alkoxycarbonyl(C₁-C₅)alkyloxy-, Di-(C₁-C₅)alkylamino-, 1-Imidazolyl-, Trifluormethyl- oder Cyangruppe sein können, oder R₁ einen Naphtylrest oder einen gesättigten oder ungesättigten, heterocyclischen Fünfring mit 1-4 Heteroatomen oder einen gesättigten oder ungesättigten, heterocyclischen Sechsring mit 1-5 Heteroatomen darstellt, wobei die Heteroatome gleich oder verschieden sein können und Sauerstoff, Schwefel oder Stickstoff bedeuten und gewünschtenfalls an einem oder mehreren Stickstoffatomen ein Sauerstoffatom tragen können, und die Fünf- oder Sechsringe gegebenenfalls durch eine oder mehrere C₁-C₆)Alkyl-, (C₁-C₆)Alkoxy-, (C₁-C₆)Alkylmercapto-, Hydroxy-, Nitro-, Amino-, Halogen- oder Cyangruppen substituiert oder mit einem Phenylring zu einem Bicyclus kondensiert sein können,
oder für den Fall, daß X eine Bindung darstellt, R₁ neben den oben genannten Gruppen auch ein Wasserstoffatom, eine (C₁-C₁₀)Alkyl-, (C₃-C₇)Cycloalkyl-, (C₁-C₁₀)Alkenyl-, (C₃-C₇)Cycloalkenyl-, (C₁-C₆)Alkoxy-(C₁-C₆)alkyl-, Carboxy(C₁-C₆)alkyl-, (C₁-C₆)Alkoxycarbonyl(C₁-C₆)alkyl-, Amino(C₁-C₆)alkyl-, Hydroxy(C₁-C₆)alkyl-, Amino-, (C₁-C₆)Alkylamino-, (C₁-C₆)Alkylcarbonylamino, Formylamino-, (C₁-C₆)Alkylsulfonylaminogruppe oder eine Alkinylgruppe mit bis zu 10 C-Atomen bedeutet,
R₂ eine (C₁-C₆)Alkyl- oder Phenylgruppe bedeutet,
R₃ eine (C₁-C₆)Alkyl- oder Phenylgruppe bedeutet oder mit R₂ zusammen eine (C₃-C₇)Cycloalkylengruppe darstellt,
R₄ einen heterocyclischen Fünfring mit 1 - 4 Heteroatomen oder einen heterocyclischen Sechsring mit 1 - 5 Heteroatomen darstellt, wobei die Heteroatome der vorgenannten Fünf- und Sechsringe gleich oder verschieden sein können und Stickstoff, Sauerstoff oder Schwefel bedeuten und die vorgenannten Fünf- und Sechsringe gegebenenfalls durch eine oder mehrere (C₁-C₆)Alkyl-, (C₁-C₆)Alkoxy-, (C₁-C₆)Alkoxy(C₁-C₆)alkyl-, (C₁-C₆)Alkylmercapto-, Hydroxy-, Hydroxy(C₁-C₆)alkyl-, Amino-, Halogen-, Oxo- oder Cyangruppen substituiert sein können,
X eine Bindung, eine (C₁-C₄)Alkylen-, die Vinylen-, die Iminogruppe -NH- oder die Carbonylaminogruppe -CONH-bedeutet,
deren Tautomere und deren physiologisch verträgliche Salze anorganischer und organischer Säuren und Verfahren zu ihrer Herstellung, sowie diese Verbindungen enthaltende Arzneimittel.

Für den Fall, daß Verbindungen der allgemeinen Formel I hergestellt wurden, die ein Asymmetriezentrum enthalten, sind auch Gegenstand der Erfindung die optisch aktiven Formen und racemischen Gemische dieser Verbindungen.

Diese neuen Verbindungen der vorliegenden Erfindung weisen wertvolle pharmakologische Eigenschaften auf, insbesondere steigern sie die Herzkraft und/oder wirken blutdrucksenkend und/oder beeinflussen die Thrombozytenfunktion und verbessern die Mikrozirkulation.

Bedeutet R₁ einen Phenylring der allgemeinen Formel II, so kann der Alkylteil der bei R₅, R₆ und R₇ genannten Substituenten vorzugsweise 1-4 Kohlenstoffatome enthalten. Bevorzugt in diesem Sinne sind beispielsweise die Methansulfonyloxy-, Ethansulfonyloxy-, n-Propansulfonyloxy-, Isopropansulfonyloxy-, Trifluormethansulfonyloxy-, Methylsulfenylmethyl-, Ethylsulfenylmethyl-, n-Propylsulfenylmethyl-, Methylsulfinylmethyl-, Ethylsulfinylmethyl-, Methylsulfonylmethyl-, Ethylsulfonylmethyl-, n-Propylsulfonylmethyl-, Methansulfonylamino-, Ethansulfonylamino-, n-Propansulfonylamino-, Trifluormethansulfonylamino-, N-Methyl-methansulfonylamino-, N-Ethyl-methansulfonylamino-, N-Methyl-ethansulfonylamino-, N-Ethyl-ethansulfonylamino-, N-Isopropyl-ethansulfonylamino-, N-Methyl-n-propansulfonylamino-, N-n-Propyl-n-propansulfonylamino-, N-Methyl-trifluormethansulfonylamino-, N-Ethyl-trifluormethansulfonylamino-, N-Isopropyltrifluormethansulfonylamino-, Methoxycarbonyl-, Ethoxycarbonyl-, Propoxycarbonyl-, Isopropoxycarbonyl-, Methylaminocarbonyl-, Ethylaminocarbonyl-, Dimethylaminocarbonyl, Di-n-propylaminocarbonyl-, N-Methyl-ethylaminocarbonyl-, Trifluormethyl-, Methylaminosulfonyl-, Ethylaminosulfonyl-, n-Propylaminosulfonyl-, n-Butylaminosulfonyl-, Dimethylaminosulfonyl-, Diethylaminosulfonyl-, Di-n-propylaminosulfonyl-, N-Methyl-isopropylaminosulfonyl-, Acetylamino-, Propionylamino-, Methylcarbonylamino-, Ethylaminocarbonylamino- oder Propylaminocarbonylaminogruppe, eine Methyl-, Ethyl-, Propyl-, Methoxy-, Ethoxy-, Propyloxy-, Allyloxy-, 2-Butenyloxy-, 3-Butenyloxy-, Propargyloxy-, 2-Butinyloxy-, 3-Butinyloxy-, Cyanmethyloxy-, Cyanethyloxy-, Methoxycarbonylmethyloxy-, Methoxycarbonylethyloxy-, Methylmercapto-, Ethylmercapto-, Methylsulfinyl-, Ethylsulfinyl-, Methylsulfonyl oder Ethylsulfonylgruppe.

Bei Sulfonylgruppen, die durch cyclische Iminogruppen substituiert sein können, sind bevorzugt die Morpholino-, Thiomorpholino-, Pyrrolidino-, Piperidino- und Hexamethyleniminosulfonylgruppen.

Insbesondere sind bevorzugt für R₅ Wasserstoff, eine (C₁-C₂)Alkylsulfonyloxy-, Trifluormethylsulfonyloxy-, (C₁-C₂)Alkylsulfenylmethyl-, (C₁-C₂)Alkylsulfinylmethyl-, (C₁-C₂)Alkylsulfonylmethyl-, (C₁-C₂)Alkylsulfonylamino-, N-(C₁-C₂)Alkyl(C₁-C₂)alkylsulfonylamino-, Trifluormethylsulfonylamino-, oder N-(C₁-C₂)Alkyltrifluormethylsulfonylaminogruppe, eine durch eine Hydroxy-, (C₁-C₂)Alkoxy-, Amino-, (C₁-C₂)Alkylamino- oder Di(C₁-C₂)Alkylaminogruppe substituierte Carbonylgruppe oder eine durch eine Amino-, Di(C₁-C₂)Alkylamino- oder Morpholinogruppe substituierte Sulfonylgruppe, eine Nitro-, Cyan- oder (C₁-C₄)Alkylaminosulfonylgruppe, eine (C₁-C₂)Alkylcarbonylamino-, Aminocarbonylamino- oder N-(C₁-C₂)Alkylaminocarbonylaminogruppe, eine (C₁-C₂)Alkylmercapto-, (C₁-C₂)Alkylsulfinyl- oder (C₁-C₂)Alkylsulfonylgruppe, eine Halogen-, Amino-, Hydroxy-, Di(C₁-C₃)alkylamino-, (C₁-C₃)Alkyl-, (C₁-C₃)Alkoxy-, (C₂-C₃)Alkenyloxy- oder (C₂-C₃)Alkinyloxygruppe , eine Cyanmethyloxy- oder Methoxycarbonylmethyloxygruppe, die Trifluormethylgruppe oder die 1-Imidazolylgruppe,
für R₆ Wasserstoff, eine (C₁-C₃)Alkylgruppe, eine (C₁-C₂)Alkoxy- oder Di(C₁-C₂)alkylaminogruppe oder ein Halogenatom und
für R₇ Wasserstoff oder die Methoxygruppe.

Der Phenylteil kann 1 bis 3 der genannten Substituenten tragen.

Bevorzugte monosubstituierte Phenylverbindungen sind die Hydroxy-, C₁-C₃ Alkyl-, C₁-C₃ Alkoxy-, Allyloxy-, Propargyloxy-, Cyanmethyloxy-, Methoxycarbonylmethyloxy-, Halogen-, Nitro-, Cyan-, Aminocarbonyl-, Methoxycarbonyl-, Amino-, C₁-C₃ Dialkylamino-, C₁-C₃ Alkylmercapto-, C₁-C₃ Alkylsulfinyl-, C₁-C₃ Alkylsulfonyl-, C₁-C₃ Alkylsulfonyloxy- und die 1-Imidazolyl-phenyle, wobei der Substituent in 2-, 3- oder 4-Stellung stehen kann.

Bevorzugte disubstituierte Phenyle enthalten als Substituenten eine Alkansulfonyloxy-, Trifluormethylsulfonyloxy-, Alkylsulfenylmethyl-, Alkylsulfinylmethyl-, Alkylsulfonylmethyl-, Alkylsulfonylamino-, N-Alkyl-alkylsulfonylamino-, Trifluormethylsulfonylamino- oder N-Alkyl-trifluormethylsulfonylaminogruppe, eine durch eine Hydroxy-, Alkoxy-, Amino-, Alkylamino- oder Dialkylaminogruppe substituierte Carbonylgruppe oder eine durch eine Amino-, Dialkylamino- oder Morpholinogruppe substituierte Sulfonylgruppe, eine Alkylaminosulfonyl-, Alkylcarbonylamino-, Aminocarbonylamino- oder N-Alkyl-aminocarbonylaminogruppe, eine Hydroxy-, Alkyl-, Alkoxy-, Allyloxy-, Propargyloxy-, Cyanmethyloxy-, Methoxycarbonylmethyloxy-, Cyan-, Halogen-, Nitro-, Amino-, Dialkylamino-, Alkylmercapto-, Alkylsulfinyl-, Alkylsulfonyl- oder eine 1-Imidazolylgruppe, wobei die beiden Substituenten gleich oder verschieden sein können und in 2,3-, 2,4-, 2,5-, 2,6-, 3,4- und 3,5-Stellung, bevorzugt jedoch in 2,4-, 2,5- und 3,4-Stellung stehen können und die vorgenannten Alkylreste, allein oder in Kombination mit anderen Resten, 1-3 C-Atomen aufweisen.

Bevorzugter trisubstituierter Phenylrest ist der 3,4,5-Trimethoxyphenylrest.

Bedeutet R₁ einen heterocyclischen Fünfring mit 1-4 Heteroatomen oder einen heterocyclischen Sechsring mit 1-5 Heteroatomen, wobei die Heteroatome der vorgenannten Fünf- oder Sechsringe gleich oder veschieden sein können und Stickstoff, Sauerstoff oder Schwefel bedeuten und gegebenenfalls an einem oder mehreren Stickstoffatomen ein Sauerstoff tragen können, so sind in diesem Sinne bevorzugt der Pyrrol-, Furan-, Thiophen-, Pyrazol-, Imidazol-, Thiazol-, Isothiazol-, Oxazol-, Isoxazol-, Triazol-, Tetrazol-, Thiadiazol-, Oxadiazol-, Pyrazin-, N,N -Dioxypyrazin-, Pyrimidin-, N,N -Dioxy-pyrimidin-, Pyridazin-, Oxazin-, Thiazin-, Triazin-, Tetrazin-, Pyridin-, N-Oxypyridin-, Piperidin-, Piperazin-, Morpholin- und Thiomorpholinrest.

Alkyl-, Alkoxy- und Alkylmercapto-Substituenten in den heterocyclischen Fünf- und Sechsringen enthalten vorzugsweise 1-4 Kohlenstoffatome Bevorzugt ist der Methyl-, Ethyl-, Methoxy-, Ethoxy-, Methylmercapto- und Ethylmercaptorest. Unter Halogen ist Fluor, Chlor und Brom, vorzugsweise Chlor zu verstehen.

Sind die heterocyclischen Fünf- und Sechsringe mit einem, Phenylring kondensiert, so sind bevorzugt der Indol-, Indazol-, Benzimidazol-, Chinolin-, Isochinolin-, Cinnolin-, Phthalazin-, Chinazolin-, Chinoxalin-, Benzofuran-, Benzothiophen-, Benzoxazol-, Benzisoxazol-, Benzothiazol- und der Benzisothiazolrest, außerdem der Naphthylrest.

Bedeutet X eine Bindung und R₁ einen (C₁-C₁₀)Alkyl-, oder einen (C₁-C₁₀)Alkenylrest oder eine Alkinylgruppe mit bis zu 10 C-Atomen, so sind darunter gerade oder verzweigte Ketten zu verstehen. Bevorzugt in diesem Sinne ist der Methyl-, Ethyl-, Propyl-, Butyl-, Pentyl-, Hexyl-, Vinyl-, Propenyl- und Propinylrest. Bedeutet X eine Bindung und R₁ einen (C₃-C₇)Cycloalkyl- oder einen (C₃-C₇)Cycloalkenylrest, ist in diesem Sinne der Cyclopropyl-, Cyclobutyl-, Cyclo-pentyl-, Cyclohexyl-, Cyclopentenyl- und Cyclohexenylrest bevorzugt. Bedeutet X eine Bindung und R₁ einen (C₁-C₆)-Alkoxy(C₁-C₆)alkyl-, Carboxy(C₁-C₆)alkyl-, (C₁-C₆)Alkoxy-carbonyl(C₁-C₆)alkyl-, Hydroxy(C₁-C₆)alkyl-, Amino(C₁-C₆)-alkyl, (C₁-C₆)Alkylamino-, oder einen (C₁-C₆)Alkyl-carbonylaminorest so ist in diesem Sinne der Ethoxymethyl-, Methoxyethyl-, Ethoxyethyl-, Carboxymethyl-, Carboxypropyl-, Carboxybutyl-, Methoxycarbonylmethyl-, Methoxycarbonylethyl-, Methoxycarbonylpropyl-, Ethoxycarbonylmethyl-, Ethoxycarbonylethyl-, Ethoxycarbonylpropyl-, Propoxycarbonylethyl-, Aminomethyl-, Aminoethyl-, Aminopropyl-, Aminobutyl-, Hydroxyethyl-, Hydroxyethyl-, Hydroxypropyl-, Hydroxybutyl-, Methylamino-, Ethylamino-, Propylamino-, Butylamino-, Acetylamino-, Propionylamino- oder der Methylsulfonylaminorest bevorzugt.

Unter Halogen sind Fluor, Chlor und Brom, vorzugsweise jedoch Chlor zu verstehen.

Bedeuten in der allgemeinen Formel I R₂ und R₃ (C₁-C₆)Alkylgruppen, so sind darunter gerade oder verzweigte Ketten zu verstehen. Bevorzugt in diesem Sinne sind die Methyl-, Ethyl-, Propyl- und die Butylgruppe.

Bilden R₂ und R₃ zusammen mit dem C-Atom, an das sie gebunden sind, einen (C₃-C₇)Ring, so sind der Cyclopropan-, Cyclobutan-, Cyclopentan- und der Cyclohexanring bevorzugt.

Bedeutet R₄ einen heterocyclischen Fünfring oder Sechsring mit 1 - 4 bzw. 1 - 5 gleichen oder verschiedenen Heteroatomen, wie Stickstoff, Sauerstoff oder Schwefel, und sind diese Fünf- und Sechsringe gegebenenfalls durch eine oder mehrere (C₁-C₆)Alkyl-, (C₁-C₆)Alkoxy-, (C₁-C₆)Alkoxy(C₁-C₆)alkyl-, (C₁-C₆)Alkylmercapto-, Hydroxy-, Hydroxy(C₁-C₆)alkyl-, Amino-, Halogen-, Oxo- oder Cyangruppen substituiert, so sind in diesem Sinne bevorzugt der 3-Oxo-2,3-dihydro-6-pyridazinyl-, 5-(C₁-C₆)Alkyl-3-oxo-2,3-dihydro-6-pyridazinyl-, 3-Oxo-2,3,4,5-tetrahydro-6-pyridazinyl-, 5-(C₁-C₆)Alkyl-3-oxo-2,3,4,5-tetrahydro-6-pyridazinyl-, 5-Hydroxy(C₁-C₆)alkyl-3-oxo-2,3,4,5-tetrahydro-6-pyridazinyl-, 3-Cyan-6-(C₁-C₆)alkyl-2-oxo-1,2-dihydro-5-pyridinyl-, 6-(C₁-C₆)Alkyl-2-oxo-1,2-dihydro-5-pyridinyl-, 3-Amino-2-oxo-1,2-dihydro-5-pyridinyl-, 3-Amino-6-(C₁-C₆)alkyl-2-oxo-1,2-dihydro-5-pyridinyl-, 3-Hydroxy-6-(C₁-C₆)alkyl-2-oxo-1,2-dihydro-5-pyridinyl-, 3-Oxo-2H-3,4-dihydro-1,4-thiazin-6-yl-, 6-Oxo-1,6-dihydro-1,2,4-triazin-3-yl-, 6-Oxo-1,4,5,6-tetrahydro-1,2,4-triazin-3-yl-, 5-Oxo-4,5-dihydro-6H-1,3,4-oxadiazin-2-yl, 5-Oxo-4,5-dihydro-6H-1,3,4-thiadiazin-2-yl-, 3-Oxo-2,3-dihydro-1,2,4-triazin-6-yl-, 3-Oxo-2,3,4,5-tetrahydro-1,2,4-triazin-6-yl, 2-Oxo-2,3-dihydro-6H-1,3,4-oxadiazin-5-yl-, 2-Oxo-2,3-dihydro-6H-1,3,4-thiadiazin-5-yl-, 5-(C₁-C₆)Alkyl-3-oxo-2,3,4,5-tetrahydro-1,2,4-triazin-6-yl-, 2-Oxo-1,2-dihydro-5-pyrimidinyl-, 4-(C₁-C₆)Alkyl-2-oxo-1,2-dihydro-5-pyrimidinyl-, 2-Oxo-1,2-dihydro-5-pyrazinyl, 3-(C₁-C₆)Alkyl-2-oxo-1,2-dihydro-5-pyrazinyl-, 6-(C₁-C₆)-Alkyl-2-oxo-1,2-dihydro-5-pyrazinyl-, 4,4-Di(C₁-C₆)alkyl-5-oxo-4,5-dihydro-3-pyrazolyl-, 2-Oxo-4-pyrrolidinyl-, 3-(C₁-C₆)Alkyl-2-oxo-4-pyrrolidinyl-, 5-Oxo-4,5-dihydro-1,2,4-triazol-3-yl-, 4-(C₁-C₆)Alkyl-5-oxo-4,5-dihydro-1,2,4-triazol-3-yl-, 2-Oxo-2,3-dihydro-4(5)-imidazolyl- oder 5(4)-(C₁-C₆)Alkyl-2-oxo-2,3-dihydro-4(5)-imidazolylrest.

Bedeutet in der allgemeinen Formel I X eine (C₁-C₄)Alkylengruppe, so ist in diesem Sinne ist die Methylen- und Ethylengruppe bevorzugt.

Besonders bevorzugte Verbindungen sind Verbindungen der allgemeinen Formel I
in der
R₁ den Phenylrest der allgemeinen Formel II bedeutet, in der R₅ ein Wasserstoffatom, die Methansulfonyloxy-, Trifluormethansulfonyloxy-, Methansulfonylamino-, Trifluormethansulfonylamino-, Methylmercapto-, Methylsulfinyl-, Methylsulfonyl-, Hydroxy-, Methyl-, Methoxy-, Propargyloxy-, Trifluormethyl- oder die 1-Imidazolylgruppe,
R₆ Wasserstoff, die Methyl-, Methoxy-, Dimethylamino- oder Chlorgruppe,
R₇ Wasserstoff oder die Methoxygruppe bedeutet oder
R₁ den Pyrrol-, Furan-, Thiophen-, Pyrazol-, Imidazol-, Isothiazol-, Thiazol-, Oxazol-, Triazol-, Tetrazol-, Thiadiazol-, Isoxazol-, Oxadiaiol-, Pyridin-, N-Oxy-pyridin-, Pyrazin-, N,N -Dioxypyrazin, Pyrimidin-, N,N -Dioxypyrimidin-, Pyridazin-, Oxazin-, Thiazin-, Triazin-, Tetrazin-, Piperidin-, Piperazin-, Morpholin- oder Thiomorpholinrest darstellt sowie deren Methyl-, Ethyl-, Methoxy-, Ethoxy-, Methylmercapto-, Ethylmercapto- und Chlor-substituierten Derivate oder den Indol-, Indazol-, Chinolin- oder Isochinolinrest bedeutet oder einen Naphthylrest darstellt oder
R₁ für den Fall, daß X eine Bindung oder die Iminogruppe -NH- darstellt, neben den genannten Gruppen auch ein Wasserstoffatom, die Methyl-, Ethyl-, Propyl-, Butyl-, Pentyl-, Hexyl-, Propenyl-, Cyclopentyl-, Cyclohexyl-, Methylamino-, Amino-, Acetamido- oder Formamidogruppe bedeutet,
R₂ und R₃ gleich sind und Methylgruppen bedeuten oder R₂ und R₃ zusammen mit dem Kohlenstoffatom, an das sie gebunden sind, eine Cyclopentanring oder Cyclohexanring bilden,
R₄ den 3-Oxo-2,3,4,5-tetrahydro-6-pyridazinyl-, 5-Methyl-3-oxo-2,3,4,5-tetrahydro-6-pyridazinyl-, 5-Hydroxymethyl-3-oxo-2,3,4,5-tetrahydro-6-pyridazinyl-, 3-Oxo-2,3-dihydro-6-pyridazinyl-, 3-Cyan-6-methyl-2-oxo-1,2-dihydro-5-pyridinyl-, 3-oxo-2,3,4,5-tetrahydro-1,2,4-triazin-6-yl, 2-Oxo-4,5-dihydro-6H-1,3,4-oxadiazin-5-yl-, 6-Oxo-1,4,5,6-tetrahydro-1,2,4-triazin-3-yl-, 5-Oxo-4,5-dihydro-6H-1,3,4-oxadiazin-2-yl-, 2-Oxo-1,2-dihydro-5-pyrimidinyl-, 2-Oxo-1,2-dihydro-5-pyrazinyl-, 4,4-Dimethyl-5-oxo-4,5-dihydro-3-pyrazolyl-, 2-Oxo-4-pyrrolidinyl-, 4-Methyl-5-oxo-4,5-dihydro-1,2,4-triazol-3-yl- oder den 5(4)-Methyl-2-oxo-2,3-dihydro-4(5)-imidazolylrest und
X einen Valenzstrich, die Vinylengruppe, die Methylengruppe, die Iminogruppe oder Carbonylaminogruppe bedeutet.

Die Verbindungen der allgemeinen Formel I können nach literaturbekannten Verfahren für die Indol-Synthese hergestellt werden.

Vergleiche hierzu:
a) P.L. Julian, E.W. Meyer und H.C. Printy, in R.C. Elderfield (Ed.), Heterocyclic Compounds, Vol. 1, S. 1-231, John Wiley and Sons, N.Y. 1952
b) R.K. Brown, in W.J. Houlihan (Ed.), Heterocyclic Compounds, Vol. 25, Part I, S. 227-537, John Wiley and Sons, N.Y. 1972.

Besonders vorteilhaft ist der in Schema 1 gezeigte Syntheseweg.

Wie aus Schema 1 ersichtlich lassen sich die literaturbekannten oder nach literaturbekannten Vergleichsverfahren hergestellten Verbindungen der allgemeinen Formel III, in der R₄ die angegebene Bedeutung hat, diazotieren, und das Diazoniumsalz zum Hydrazin IV reduzieren. Durch Umsetzung dieser Hydrazine mit Verbindungen der allgemeinen Formel V

R₁-X-COCHR₂R₃ (V)

in der
R₁,R₂,R₃ und X die oben genannte Bedeutung haben, gelangt man zu den Hydrazonen VI, die durch eine Fischer-Indol Synthese zu Verbindungen der allgemeinen Formel I cyclisiert werden können. Andererseits lassen sich die Hydrazone der allgemeinen Formel VI auch dadurch erhalten, daß man das Diazonium-Salz der Amine III in einer Japp-Klingemann-Reaktion mit Verbindungen der allgemeinen Formel VII 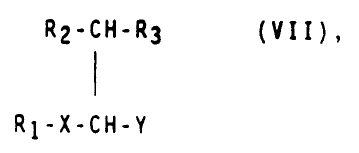
in der 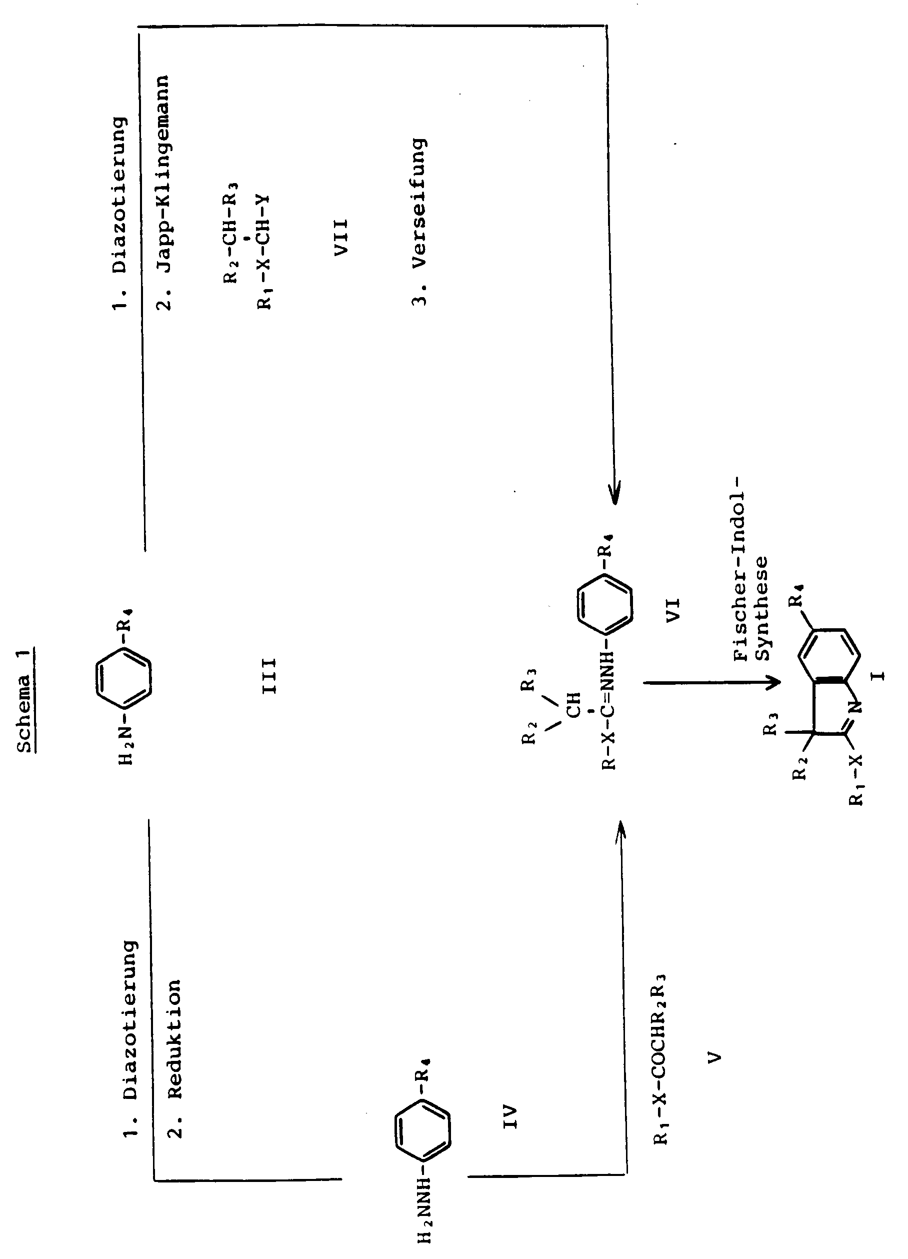
R₁,R₂,R₃ und X die angegebene Bedeutung haben und Y ein die Methingruppe aktivierender Rest ist, umsetzt. Dieser Rest kann z.B. ein Aldehyd, Keton, Ester, Carbonsäure oder Nitril sein. Die im Reaktionsgemisch intermediaer anfallende Azoverbindung wird ohne Isolierung direkt zum Hydrazon verseift.

Die Verbindungen der Formel VI sind mit Ausnahme der Verbindung 6-(4-sec. Butylidenhydrazinophenyl)-5-methyl-4,5-dihydro-1,2,4-triazin-3(2H)-on, die in EP-A-0 122 494 aüf Seite 39 erwähnt wird, neu und ebenfalls Gegenstand der Erfindung.

Die Diazotierung der Amine III wird vorzugsweise unter neutralen oder sauren Bedingungen in einem polaren Lösungsmittel wie Wasser, Methanol, Ethanol, Eisessig, Salzsäure, Schwefelsäure oder Phosphorsäure bei Temperaturen zwischen -70°C und 50°C vorzugsweise jedoch zwischen -5°C und 10°C durchgeführt.

Zur Diazotierung kommen vorwiegend anorganische Salze oder organische Ester der salpetrigen Säure in Frage wie z.B. NaNO₂, KNO₂ oder Amylnitrit.

Die Reduktion der Diazoniumsalze wird überwiegend in den oben genannten Lösungsmitteln, in denen die Diazotierung ausgeführt wurde, bei Temperaturen zwischen -50°C und dem Siedepunkt des Lösungsmittels vorgenommen, vorzugsweise jedoch zwischen 0°C und 80°C, wobei als Reduktionsmittel Alkalisulfite, Schwefeldioxid, Dithionite, Zinn(II)chlorid, Zinkstaub, Eisen, Natriumamalgam, Triphenylphosphin, Endiole oder auch eine elektrochemische Reduktion in Frage kommen.

Die Umsetzung der Hydrazine mit Verbindungen der allgemeinen Formel V kann in Lösungsmitteln wie Wasser, Alkohol, Benzol, Toluol, Dioxan, DMF, Diethylether oder THF bei Temperaturen zwischen -80°C bis zum Siedepunkt des verwendeten Lösungsmittel durchgeführt werden. Vorteilhaft ist auch der Zusatz einer anorganischen oder organischen Säure wie Salzsäure, Schwefelsäure, Phosphorsäure oder Essigsäure.

Die Japp-Klingemann-Reaktion wird vorteilhaft in den Lösungsmitteln durchgeführt, in denen die bereits oben beschriebene Diazotierung durchgeführt werden kann. Dies sind also insbesondere Wasser, Methanol, Ethanol, Eisessig, Salzsäure, Schwefelsäure oder Phosphorsäure bei Temperaturen zwischen -50°C bis 80°C vorzugsweise jedoch zwischen 0°C und 25°C. Die nachfolgende Verseifung kann thermisch oder nach Zusatz einer Base oder Säure wie z.B. Natronlauge, Kalilauge, Salzsäure, Schwefelsäure, Phosphorsäure oder Eisessig bei Temperaturen bis zum Siedepunkt des Lösungsmittels durchgeführt werden.

Die Fischer-Indol-Synthese der Hydrazone VI wird ohne Lösungsmittel oder in einem Solvens wie Alkohol, Nitrobenzol, Essigsäure, Xylol, Cumol, Toluol thermisch oder in Gegenwart eines sauren Katalysators, der jedoch auch Lösungsmittel sein kann, durchgeführt, wobei Salzsäure, Schwefelsäure, Phosphorsäure, Polyphosphorsäure, Eisessig, Ameisensäure, Zink(II)chlorid, Bortrifluorid, Kationenaustauscher, Sulfosalicylsäure oder Polyphosphatester in Frage kommen bei Temperaturen zwischen 0°C und dem Siedepunkt des verwendeten Lösungsmittels.

Die Hydrazone der allgemeinen Formel VI lassen sich gegebenenfalls auch aus den Aminen III über die Sydnone X nach Schema 2 herstellen. 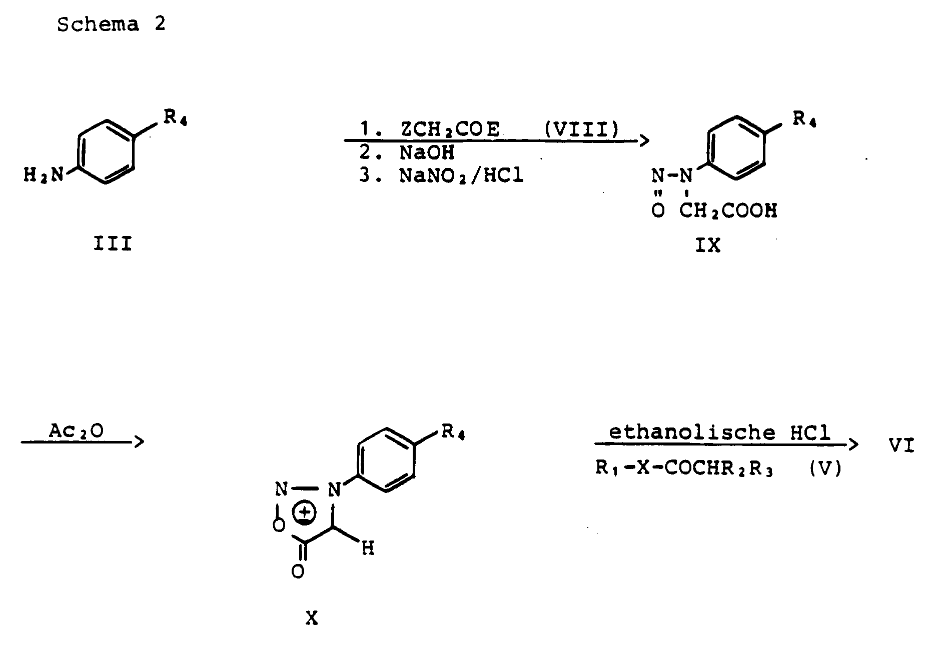
Die Umsetzung von Aminen III mit Halogenessigestern VIII, in denen Z ein Halogen wie F, Cl, Br oder J, vorzugsweise jedoch Br, bedeutet, wird vorteilhaft in polaren oder unpolaren Lösungsmittel wie z.B. Methylenchlorid, Toluol, Dioxan, Alkoholen oder Dimethylformid bei Temperaturen zwischen -50°c und dem Siedepunkt des Lösungsmittels, vorzugsweise zwischen 25°C und 100°C, durchgeführt.

Die so erhaltenen Ester können nach wohlbekannten Verfahren z.B. mit anorganischen Basen wie z.B. Natriumhydroxid, Kaliumhydroxid, Natriumhydrogencarbonat oder Kaliumhydrogencarbonat in protischen Lösungsmitteln wie Wasser oder Alkohol oder mit anorganischen oder organischen Säuren wie Salzsäure, Schwefelsäure, Eisessig, Phosphorsäure oder Polyphosphorsäure gegebenenfalls unter Zugabe eines Lösungsmittels wie Wasser oder Alkohol verseift werden.

Die Nitrosierung der erhaltenen Säuren zu den Verbindungen der allgemeinen Formel IX wird vorzugsweise unter neutralen oder sauren Bedingungen in einem polaren Lösungsmittel wie Wasser, Methanol, Ethanol, Eisessig, Salzsäure, Schwefelsäure oder Phosphorsäure bei Temperaturen zwischen -70°C und 50°C vorzugsweise jedoch zwischen -5°C und 10°C durchgeführt. Zur Nitrosierung kommen vorwiegend anorganische Salze oder organische Ester der salpetrigen Säure in Frage wie z.B. NaNO₂, KNO₂ oder Amylnitrit.

Die Umsetzung der N-Nitroso-carbonsäuren IX zu den Sydnonen X gelingt in inerten Lösungsmittel wie z.B. Dioxan, Diethylether, Tetrahydrofuran, Toluol mit wasserentziehenden Reagenzien wie z.B. Acetanhydrid, Propionsäureanhydrid, Schwefelsäure, Phosphorpentoxid, PCl₅ oder PCl₃ bei Temperaturen zwischen -50°C und dem Siedepunkt des Lösungsmittels, vorteilhaft jedoch zwischen 25°C und 100°C.

Die Sydnone X lassen sich unter sauren Bedingungen in die Hydrazine IV zerlegen, die in situ mit den Ketonen V zu den Hydrazonen VI abgefangen werden. Als Säuren für die Verseifung der Sydnone kommen Salzsäure, Schwefelsäure, Phosphorsäure, Polyphorphorsäure oder organische Säuren wie Eisessig bei Temperaturen zwischen -70°C und 100°C vorzugsweise zwischen 0°C und 70°C in Betracht.

Bedeutet R₄ in Verbindungen der allgemeinen Formel I eine 3-Oxo-2,3,4,5-tetrahydro-6-pyridazinyl- oder 5-Methyl-3-oxo-2,3,4,5-tetrahydro-6-pyridazinylrest, so lassen sich diese Verbindungen der allgemeinen Formel I auch dadurch herstellen, daß man Verbindungen der allgemeinen Formel XI 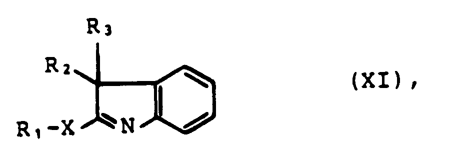
in der R₁,R₂,R₃ und X die angegebene Bedeutung haben, mit Säurechloriden der Formel XII

R₈OOCCH₂CHR₉COCl (XII),

oder Carbonsäureanhydriden der Formel XIII 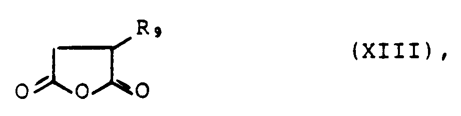
in denen R₈ Alkylreste mit 1-6 C-Atomen darstellen und R₉ Wasserstoff oder die Methylgruppe ist, zu Verbindungen der allgemeinen Formel XIV oder deren R₈-Ester 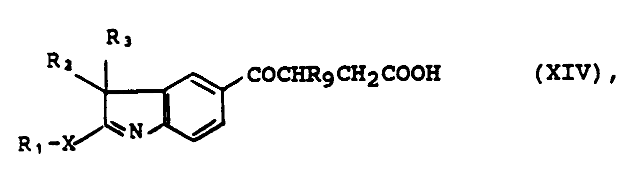
umsetzt.
Die Verbindungen der allgemeinen Formel XIV können mit Hydrazinen zu Verbindungen der allgemeinen Formel I, in denen R₁,R₂,R₃ und X die angegebene Bedeutung haben und R₄ einen 3-Oxo-2,3,4,5-tetrahydro-6-pyridazinyl- oder 5-Methyl-3-oxo-2,3,4,5-tetrahydro-6-pyridazinylrest bedeutet, cyclisiert werden.

Die Umsetzung von Verbindungen der Formel XI mit Verbindungen der Formel XII oder XIII führt man in einem Lösungsmittel wie Kohlendisulfid, Methylenchlorid, Dichlorethan oder Nitrobenzol in Gegenwart eines Überschußes einer Lewissäure wie Aluminiumchlorid oder Aluminiumbromid bei Temperaturen zwischen 0°C und 150°C, vorzugsweise beim Siedepunkt des Lösungsmittels oder in Gegenwart eines großen Überschußes (bis 10 mol) Aluminiumchlorid in Dimethylformamid bei Temperaturen zwischen 0°C und 150°C durch.

Die Cyclisierung von Verbindungen der Formel XIV erfolgt vorzugsweise in einem Lösungsmittel wie Ethanol, Isopropanol oder in einer Mischung wie Isopropanol/Wasser in Gegenwart von 1-5 mol Hydrazinhydrat, vorzugsweise mit 1-2 mol Hydrazinhydrat, bei Temperaturen zwischen Raumtemperatur und dem Siedepunkt des Lösungsmittels oder Lösungsmittelgemisches.

Verbindungen der allgemeinen Formel I können auch nachträglich in eine andere Verbindung der Formel I umgewandelt werden.
Dies trifft zum Beispiel zu:
a) für die Oxidation eines Fünf- oder Sechsringes mit einem oder mehreren Stickstoffatomen zu den entsprechenden N-Oxiden. Die Oxidation wird zweckmäßig mit einem oder mehreren Aequivalenten des verwendeten Oxidationsmittels durchgeführt, z.B. mit Wasserstoffperoxid in Eisessig, Trifluoressigsäure oder in Ameisensäure bei 20-100°C oder in Aceton bei 0-60°C, mit einer Persäure wie Perameisensäure oder m-Chlorperbenzoesäure in Eisessig, Trifluoressigsäure, Methylenchlorid oder Chloroform bei Temperaturen zwischen 0 und 60°C.
b) für die Herstellung von Verbindungen der allgemeinen Formel I, in der R₁ einen Rest der allgemeinen Formel II bedeutet und R₅ eine (C₁-C₅)Alkysulfinyl-, (C₁-C₅)Alkylsulfonyl-, (C₁-C₅)Alkylsulfinylmethyl- oder (C₁-C₅)Alkylsulfonylmethylgruppe darstellt, durch nachträgliche Oxidation einer Verbindung der allgemeinen Formel XV, 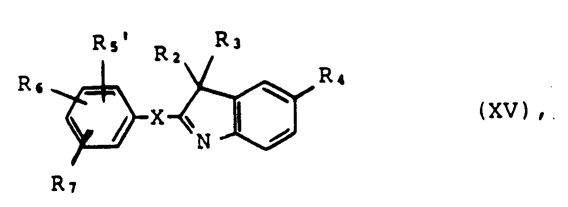 in der
   R₂,R₃,R₄,R₆,R₇ und X wie eingangs definiert sind und R₅ʹ eine Alkylmercapto- oder Alkylsulfenylmethylgruppe mit jeweils 1-3 Kohlenstoffatomen im Alkylteil ist. Die Oxidation wird vorzugsweise in einem Lösungsmittel oder Lösungsmittelgemisch, z.B. Wasser, Wasser/Pyridin, Aceton, Eisessig, verdünnter Schwefelsäure oder Trifluoressigsäure, je nach dem verwendeten Oxidationsmittel zweckmäßigerweise bei Temperaturen zwischen -80°C und 100°C durchgeführt.
   Zur Herstellung einer Alkylsulfinyl- oder Alkylsulfinylmethylverbindung der allgemeinen Formel I wird die Oxidation zweckmäßigerweise mit einem Aequivalent des verwendeten Oxidationsmittels durchgeführt, z.B. mit Wasserstoffperoxid in Eisessig, Trifluoressigsäure oder Ameisensäure bei 0 bis 20°C oder in Aceton bei 0 bis 60°C, mit einer Persäure wie Perameisensäure in Eisessig oder Trifluoressigsäure bei 0 bis 50°C oder mit m-Chlorperbenzoesäure in Methylenchlorid oder Chloroform bei -20 bis 60°C, mit Natriummetaperjodat in wässrigem Methanol oder Ethanol bei -15 bis 25°C, mit Brom in Eisessig oder wässriger Essigsäure, mit N-Bromid-succinimid in Ethanol, mit tert.-Butyl-hypochlorit in Methanol bei -80 bis -30°C, mit Jodbenzodichlorid in wässrigem Pyridin bei 0 bis 50°C, mit Salpetersäure in Eisessig bei 0 bis 20°C, mit Chromsäure in Eisessig oder in Aceton bei 0 bis 20°C und mit Sulfurylchlorid in Methylenchlorid bei -70°C, der hierbei erhaltene Thioether-Chlorkomplex wird zweckmäßigerweise mit wässrigem Ethanol hydrolysiert.
   Zur Herstellung einer Alkylsulfonyl- oder Alkylsulfonylmethylverbindung der allgemeinen Formel I wird die Oxidation zweckmäßigerweise mit einem bzw. mit zwei oder mehr Aequivalenten des verwendeten Oxidationsmittels durchgeführt, z.B. Wasserstoffperoxid in Eisessig, Trifluoressigsäure oder in Ameisensäure bei 20 bis 100°C oder in Aceton bei 0 bis 60°C,. mit einer Persäure wie Perameisensäure oder m-Chlorperbenzoesäure in Eisessig, Trifluoressigsäure, Methylenchlorid oder Choroform bei Temperaturen zwischen 0 und 60°C, mit Salpetersäure in Eisessig bei 0 bis 20°C, mit Chromsäure oder Kaliumpermanganat in Eisessig, Wasser/Schwefelsäure oder in Aceton bei 0 bis 20°C.
c) für die Herstellung von Verbindungen der allgemeinen Formel I, in der R₁ einen Rest der allgemeinen Formel II bedeutet und R₅ eine (C₁-C₅)Alkansulfonyloxy-, Trifluoromethansulfonyloxy-, (C₁-C₅)Alkansulfonylamino-, N-(C₁-C₅)Alkyl-(C₁-C₅)alkansulfonylamino-, Trifluormethansulfonyl- oder N-(C₁-C₅)Alkyl-trifluormethansulfonylaminogruppe darstellt, durch die nachträgliche Umsetzung einer Verbindung der allgemeinen Formeln XVI 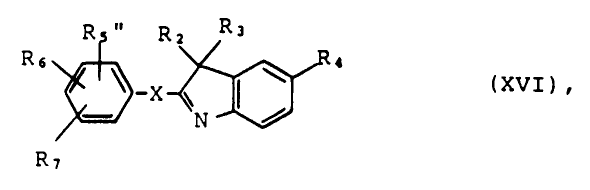 in der
   R₂,R₃,R₄,R₆,R₇ und X wie eingangs definiert sind und R₅'' eine Hydroxy-, Amino- oder N-Alkylaminogruppe mit 1-3 Kohlenstoffatomen im Alkylteil darstellt, mit einer Sulfonsäure der allgemeinen Formel XVII

   R₁₀-SO₂OH (XVII),

   in der
   R₁₀ eine Alkylgruppe mit 1-3 Kohlenstoffatomen oder eine Trifluormethylgruppe darstellt, in Gegenwart eines wasserentziehenden und/oder die Säure oder das Amin aktivierenden Mittels oder mit deren reaktionsfähigen Derivaten.
   Die Umsetzung wird zweckmäßigerweise in einem Lösungsmittel oder Lösungsmittelgemisch wie Methylenchlorid, Ether, Tetrahydrofuran, Dioxan oder Benzol gegebenenfalls in Gegenwart eines säurebindenden Mittels wie Natriumcarbonat, Triethylamin oder Pyridin, wobei die beiden letzteren gleichzeitig auch als Lösungsmittel verwendet werden können, in Gegenwart eines die Säure aktivierenden oder wasserentziehenden Mittels wie Thionylchlorid oder Phosphorpentachlorid, vorzugsweise jedoch mit einem reaktionsfähigen Derivat einer Verbindung der allgemeinen Formel XVII z.B. mit deren Anhydrid oder Halogenid wie Methansulfonsäurechlorid oder Ethansulfonsäurechlorid, vorzugsweise bei Temperaturen zwischen 0 und 100°C, z.B. bei Temperaturen zwischen Raumtemperatur und 50°C, durchgeführt.
d) für die Herstellung von Verbindungen der allgemeinen Formel I, in der R₁ einen Rest der allgemeinen Formel II bedeutet und R₅ eine durch eine Amino-, (C₁-C₅)Alkylamino- oder Di(C₁-C₅)alkylaminogruppe substituierte Carbonyl- oder Sulfonylgruppe darstellt, durch die nachträgliche Umsetzung einer Verbindung der allgemeinen Formel XVIII 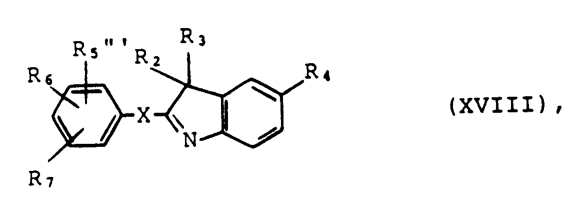 in der
   R₂,R₃,R₄,R₆,R₇ und X wie eingangs definiert sind und R₅''' eine Carboxyl- oder Hydroxysulfonylgruppe darstellt, oder einem reaktionsfähigen Derivat hiervon wie z.B. Ester oder Säurechlorid mit einem Amin der allgemeinen Formel XIX 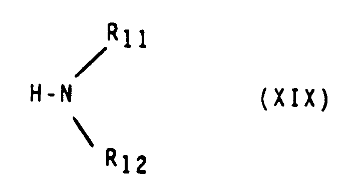 in der
   R₁₁ und R₁₂, die gleich oder verschieden sein können, Wasserstoffatome oder Alkylgruppen mit 1-5 Kohlenstoffatomen darstellen, oder mit einem reaktionsfähigen Derivat hiervon, falls R₅''' die Carboxyl- oder Hydroxysulfonylgruppe darstellt. Die Umsetzung wird zweckmäßigerweise in einem Lösungsmittel wie Methylenchlorid, Ethanol, Chloroform, Tetrachlorkohlenstoff, Ether, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureethylester, Thionylchlorid, Phosphortrichlorid, Phosphorpentoxid, N,N -Dicyclohexylcarbodiimid/N-Hydroxysuccinimid, N,N -Carbonyldiimidazol oder N,N -Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiaeren organischen Base wie Triethylamin oder Pyridin, weiche gleichzeitig als Lösungsmittel dienen können, bei Temperaturen zwischen -25 und 250°C, vorzugsweise jedoch bei Temperaturen zwischen -10°C und der Siedetemperatur des verwendeten Lösungsmittels durchgeführt, desweiteren kann während der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.
   Besonders vorteilhaft wird jedoch die Umsetzung mit einem entsprechenden Halogenid, z.B. dem Carbonsäure- oder Sulfonsäurechlorid und einem entsprechenden Amin, wobei dieses gleichzeitig als Lösungsmittel dienen kann, und bei Temperaturen zwischen 0 und 50°C durchgeführt.
e) für die Herstellung von Verbindungen der allgemeinen Formel I, in der R₁ einen Phenylring der Formel II oder einen heterocyclischen Fünf- oder Sechsring bedeutet und X die Carbonylaminogruppe darstellt, durch nachträgliche Acylierung von Verbindungen der allgemeinen Formel I, in der R₂,R₃ und R₄ die angegebene Bedeutung haben, R₁ Wasserstoff ist und X die Gruppe NH bedeutet. Diese Reaktionen werden vorzugsweise durch Umsetzung mit Carbonsäurederivaten wie Säurehalogeniden, Carbonsäureestern oder anderen aktivierten Carbonsäurederivaten wie z.B. Anhydriden, durchgeführt.
f) für die Herstellung von Verbindungen der allgemeinen Formel I, in der R₁ ein Rest der allgemeinen Formel II ist, wobei R₅ eine Carboxy-, (C₁-C₅)Alkoxycarbonyl-, Aminocarbonyl-, (C₁-C₅)Alkoxycarbonyl(C₁-C₅)alkyoxy- oder Carboxy(C₁-C₅)alkyloxygruppe bedeutet, durch nachträgliche Alkoholyse und/oder Hydrolyse von Verbindungen der allgemeinen Formel I, in der R₁ ein Rest der allgemeinen Formel II ist, wobei R₅ eine Cyan- oder Cyan(C₁-C₅)alkyloxygruppe darstellt.
   Die nachträgliche Alkoholyse und/oder Hydrolyse wird zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Wasser/Methanol, Ethanol, Wasser/Ethanol, Wasser/Isopropanol und Wasser/Dioxan bei Temperaturen zwischen -10 und 120°C, z.B. bei Temperaturen zwischen Raumtemperatur und der Siedetemperatur des Reaktionsgemisches, durchgeführt.

Ferner können die so erhaltenen Verbindungen der allgemeinen Formel I anschließend gewünschtenfalls in ihre physiologisch verträglichen Säureadditionssalze mit anorganischen oder organischen Säuren überführt werden. Als Säuren kommen hierfür beispielsweise Salzsäure, Bromwasserstoffsäure, Schwefelsäure, Phosphorsäure, Fumarsäure, Bernsteinsäure, Weinsäure, Zitronensäure, Milchsäure, Maleinsäure oder Methansulfonsäure in Betracht.

Wie bereits eingangs erwähnt weisen die neuen Verbindungen der allgemeinen Formel I, deren Tautomere und deren physiologisch verträgliche Säureadditionssalze bei einer langen Wirkungsdauer überlegene pharmakologische Eigenschaften auf, insbesondere eine blutdrucksenkende und/oder positiv inotrope Wirkung und/oder beeinflussen die Thrombozytenfunktion und verbessern die Mikrozirkulation, während die im Stand der Technik in FR-A-2 530 246 beschriebenen Indolinderivate Substanzen mit negativ inotropen und blutdrucksenkenden Eigenschaften sind.

Zur Herstellung von Arzneimitteln werden die Substanzen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die erfindungsgemäßen neuen Substanzen der allgemeinen Formel I und ihre Salze können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungsmittel, Lösungsvermittler oder Puffer enthält.

Derartige Zusätze sind z.B. Tartrat- und Citratpuffer, Ethanol, Komplexbildner (wie Ethylendiamintetraessigsäure und deren nicht toxische Salze) und hochmolekulare Polymere (wie flüssiges Polyethylenoxid) zur Viskositätsregulierung. Feste Trägerstoffe sind z.B. Stärke, Lactose, Mannit, Methylcellulose, Talkum, hochdisperse Kieselsäuren, hochmolekulare Fettsäuren (wie Staerinsäure), Gelatine, Agar-Agar, Calciumphosphat, Magnesiumstearat, tierische und pflanzliche Fette und feste hochmolekulare Polymere (wie Polyethylenglykole). Für orale Applikation geeignete Zubereitungen können gewünschtenfalls Geschmacks- und Süßstoffe enthalten.

Die erfindungsgemäßen Verbindungen werden üblicherweise in Mengen von 1-500 mg pro Tag bezogen auf 75 kg Körpergewicht appliziert. Bevorzugt ist es, 2-3 mal pro Tag 1-2 Tabletten mit einem Wirkstoffgehalt von 0.5-200 mg zu verabreichen. Die Tabletten können auch retardiert sein, wodurch nur noch 1 mal pro Tag 1-2 Tabletten mit 1-500 mg Wirkstoff gegeben werden müssen. Der Wirkstoff kann auch durch Injektion 1-8 mal pro Tag bzw. durch Dauerinfusion gegeben werden, wobei Mengen von 0.5-200 mg pro Tag normalerweise ausreichen.

Besonders bevorzugt im Sinne der vorliegenden Erfindung sind außer den in den Beispielen genannten Verbindungen die folgenden und deren Tautomere:
2-(3-Pyridyl)-3,3-dimethyl-5-(3-oxo-2,3,4,5-tetrahydro-6-pyridazinyl)-3H-indol
2-(4-Pyridazinyl)-3,3-dimethyl-5-(3-oxo-2,3,4,5-tetrahydro-6-pyridazinyl)-3H-indol
2-(2-Pyrazinyl)-3,3-dimethyl-5-(3-oxo-2,3,4,5-tetrahydro-6-pyridazinyl)-3H-indol
2-(3-Thienyl)-3,3-dimethyl-5-(3-oxo-2,3,4,5-tetrahydro-6-pyridazinyl)-3H-indol
2-(4-Thiazolyl)-3,3-dimethyl-5-(3-oxo-2,3,4,5-tetrahydro-6-pyridazinyl)-3H-indol
2-(2-Chlorphenyl)-3,3-dimethyl-5-(3-oxo-2,3,4,5-tetrahydro-6-pyridazinyl)-3H-indol
2-(3-Trifluormethylphenyl)-3,3-dimethyl-5-(3-oxo-2,3,4,5-tetrahydro-6-pyridazinyl)-3H-indol
2-(2-Hydroxyphenyl)-3,3-dimethyl-5-(3-oxo-2,3,4,5-tetrahydro-6-pyridazinyl)-3H-indol
2-(4-Aminocarbonylphenyl)-3,3-dimethyl-5-(3-oxo-2,3,4,5-tetrahydro-6-pyridazinyl)-3H-indol
2-[4-(1H-Imidazol-1-yl)phenyl-3,3-dimethyl-5-(3-oxo-2,3,4,5-tetrahydro-6-pyridazinyl)-3H-indol
2-(4-Pyridylcarbonylamino)-3,3-dimethyl-5-(3-oxo-2,3,4,5-tetrahydro-6-pyridazinyl)-3H-indol
3,3-Dimethyl-5-(3-oxo-2,3,4,5-tetrahydro-6-pyridazinyl)-3H-indol
2-(4-Pyridyl)-3,3-dimethyl-5-(3-oxo-2,3,4,5-tetrahydro-1,2,4-triazin-6-yl)-3H-indol
2-(4-Pyridyl)-3,3-dimethyl-5-(2-oxo-2,3-dihydro-6H-1,3,4-oxadiazin-5-yl)-3H-indol
2-(4-Pyridyl)-3,3-dimethyl-5-(5-oxo-4,5-dihydro-6H-1,3,4-oxadiazin-2-yl)-3H-indol
2-(4-Pyridyl)-3,3-dimethyl-5-(2-oxo-1,2-dihydro-5-pyrazinyl)-3H-indol
2-(4-Pyridyl)-3,3-dimethyl-5-(4,4-dimethyl-5-oxo-4,5-dihydro-3-pyrazolyl)-3H-indol
2-(4-Pyridyl)-3,3-dimethyl-5-(4-Methyl-5-oxo-4,5-dihydro-1,2,4-triazol-3-yl)-3H-indol
2-(4-Pyridyl)-3,3-dimethyl-5-(3-oxo-2,3-dihydro-6-pyridazinyl)-3H-indol
2-(4-Pyridyl)-3,3-dimethyl-5-(5-methyl-3-oxo-2,3-dihydro-6-pyridazinyl)-3H-indol
2-(4-Pyridyl)-3,3-diethyl-5-(5-methyl-3-oxo-2,3,4,5-tetrahydro-6-pyridazinyl)-3H-indol
2-(4-Pyridyl)-3-methyl-3-ethyl-5-(5-methyl-3-oxo-2,3,4,5-tetrahydro-6-pyridazinyl)-3H-indol
2-(4-Pyridyl)-3-methyl-3-butyl-5-(5-methyl-3-oxo-2,3,4,5-tetrahydro-6-pyridazinyl)-3H-indol
2-(4-Pyridyl)-3-methyl-3-phenyl-5-(5-methyl-3-oxo-2,3,4,5-tetrahydro-6-pyridazinyl)-3H-indol
2-(4-Pyridyl-3,3-diphenyl-5-(5-methyl-3-oxo-2,3,4,5-tetrahydro-6-pyridazinyl)-3H-indol
2 -(4-Pyridyl)-spiro[cyclopentan-1,3 -5 -(5-methyl-3-oxo-2,3,4,5-tetrahydro-6-pyridazinyl)-3 H-indol]
2 -(4-Pyridyl)-spiro[cyclohexan-1,3 -5 -(5-methyl-3-oxo-2,3,4,5-tetrahydro-6-pyridazinyl)-3 H-indol]
2 -(4-Pyridyl)-spiro[cyclopropan-1,3 -5 -(5-methyl-3-oxo-2,3,4,5-tetrahydro-6-pyridazinyl)-3 H-indol]
2 -(4-Methoxyphenyl)-spiro[cyclopentan-1,3 -5 -(5-methyl-3-oxo-2,3,4,5-tetrahydro-6-pyridazinyl)-3 H-indol]
2 -(4-Morpholino)-spiro[cyclopentan-1,3 -5 -(5-methyl-3-oxo-2,3,4,5-tetrahydro-6-pyridazinyl)-3 H-indol]

### Beispiel 1

### 2,3,3-Trimethyl-5-(3-oxo-2,3,4,5-tetrahydro-6-pyridazinyl)-3H-indol Hydrochlorid

a) 6.0 g (24.9 mmol) 6-(4-Hydrazinophenyl)-2,3,4,5-tetrahydro-pyridazin-3-on Hydrochlorid in 170 ml 50proz. Ethanol wurden bei Raumtemperatur mit 3.2 ml (29.7 mmol) Isopropylmethylketon versetzt. Nach 3 Stunden wurde abgesaugt, der Rückstand mit 50proz. Ethanol nachgewaschen, erneut in Wasser suspendiert und mit 2 N Ammoniak neutralisiert und abgesaugt. Man erhält 5.1 g Isopropylmethylketon-[4-(3-oxo-2,3,4,5-tetrahydro-6-pyridazinyl)phenylhydrazon] vom Schmp. 98-100°C.
b) 3.5 g (12.9 mmol) des Hydrazons wurden unter Stickstoff in 40 ml Polyphosphorsäure 3 Stunden bei 120°C gerührt. Der Ansatz wurde noch warm auf Eiswasser gegossen, neutralisiert, mit Dichlormethan extrahiert, getrocknet und eingeengt. Nach Reinigung über Kieselgel (Laufmittel: Dichlormethan/Ethanol 99:1) wurde der Rückstand in Ethanol gelöst, mit ethanolischer Salzsäure sauer gestellt und die Kristalle abgesaugt. Man erhält 1.9 g der Titelverbindung vom Schmp. 276°C als Hydrochlorid.

### Beispiel 2

Analog Beispiel 1 b erhält man bei Arbeiten in Eisessig bei 100°C die nachfolgenden Titelverbindungen:

| Bezeichnung | | Ausb. % | Schmelzpunkt °C (Lsg.mittel) |
|---|---|---|---|
| a) | 2-(4-Pyridyl)-3,3-dimethyl-5-(5-methyl-3-oxo-2,3,4,5-tetrahydro-6-pyridazinyl)-3H-indol aus 4-Pyridyl-isopropylketon-[4-(5-methyl-3-oxo-2,3,4,5-tetrahydro-6-pyridazinyl)phenylhydrazon] Schmp. 225-227°C | 63 | 303-05 CH₃OH |
| b) | 2-(4-Pyridyl)-3,3-dimethyl-5-(3-oxo-2,3,4,5-tetrahydro-6-pyridazinyl)-3H-indol aus 4-Pyridyl-isopropylketon-[4-(3-oxo-2,3,4,5-tetrahydro-6-pyridazinyl)phenylhydrazon] Schmp. 247-249°C | 73 | 269-70°C EtOH |

### Beispiel 3

### 2-Methylamino-3,3-dimethyl-5-(3-oxo-2,3,4,5-tetrahydro-6-pyridazinyl)-3H-indol

a) 5.2 g (29.7 mmol) 2-Methylamino-3,3-dimethyl-3H-indol wurden in 100 ml Dichlormethan vorgelegt, 16 g Aluminiumtrichlorid eingetragen und unter Eiskühlung 5.4 g (33 mmol) Bernsteinsäureethylesterchlorid zugetropft. Man rührt 4 Stunden bei 25°C, gießt auf Eis, extrahiert, trocknet und engt ein. Man erhält 8.1 g 4-Oxo-4-(2-methylamino-3,3-dimethyl-3H-indol-5-yl)buttersäureethylester vom Schmp. 90-96°C.
b) 8 g (26.5 mmol) des obigen Esters wurden mit 4 ml Hydrazin-Hydrat, 40 ml Ethanol und 0.2 ml Eisessig 2 Tage bei Raumtemperatur gerührt, anschließend abgesaugt, der Rückstand an Kieselgel (Laufmittel: Dichlormethan/mit Ammoniak gesättigtes Methanol 15:1) gereinigt und aus Ethanol/Wasser umkristallisiert. Man erhält 4.2 g der Titelverbindung vom Schmp 320-24°C.

### Beispiel 4

### 2-Phenyl-3,3-dimethyl-5-(3-oxo-2,3,4,5-tetrahydro-6-pyridazinyl)-3H-indol

3 g (12.5 mmol) 6-(4-Hydrazinophenyl)-2,3,4,5-tetrahydropyridazin-3-on Hydrochlorid, 2.4 g (16.2 mmol) alpha-Methylpropiophenon und 40 ml Eisessig wurden 8 Std. bei 100°C unter Stickstoff gerührt. Anschließend wurde der Eisessig abdestilliert, der Rückstand in Wasser suspendiert, neutralisiert und abgesaugt. Nach Umkristallisation aus Ethanol erhält man 1.6 g (40 %) der Titelverbindung vom Schmp. 200-01°C.

### Beispiel 5

Wie in Beispiel 4 beschrieben erhält man:

| Bezeichnung | | Ausb. % | Schmelzpunkt °C (Lsg.mittel) |
|---|---|---|---|
| a) | 2-(4-Methoxyphenyl)-3,3-dimethyl-5-(3-oxo-2,3,4,5-tetrahydro-6-pyridazinyl)-3H-indol | 59 | 264-65 EtOH |
| b) | 2-(4-Chlorphenyl)- 3,3-dimethyl-5-(3-oxo-2,3,4,5-tetrahydro-6-pyridazinyl)-3H-indol | 52 | 250-53 EtOH |
| c) | 2-(4-Methylphenyl)-3,3-dimethyl-5-(3-oxo-2,3,4,5-tetrahydro-6-pyridazinyl)-3H-indol | 62 | 258-60 EtOH |
| d) | 2-(4-Methylmercaptophenyl)-3,3-dimethyl-5-(3-oxo-2,3,4,5-tetrahydro-6-pyridazinyl)-3H-indol | 48 | 226-27 EtOH |
| e) | 2-(2-Thienyl)-3,3-dimethyl-5-(3-oxo-2,3,4,5-tetrahydro-6-pyridazinyl)-3H-indol | 35 | 216-17 EtOH |
| f) | 2'-Phenyl-spiro[cyclopentan-1,3'-5'-(3-oxo-2,3,4,5-tetrahydro-6-pyridazinyl)-3'H-indol] | 41 | 232-33 CH₃OH |

### Beispiel 6

### 2-(4-Pyridyl)-3,3-dimethyl-5-(3-oxo-2,3-dihydro-6-pyridazinyl)-3H-indol

1.75 g (5.5 mmol) 2-(4-Pyridyl)-3,3-dimethyl-5-(3-oxo-2,3,4,5-tetrahydro-6-pyridazinyl)-3H-indol (Bsp. 2 b) wurden mit 25 g MnO₂ in 260 ml Dioxan 20 Std. bei 90°C gerührt. Anschließend wurde abgesaugt, der Rückstand mit Methanol/Methylenchlorid ausgerührt und die vereinigten Filtrate zur Trockne gebracht. Nach Kristallisation aus Methanol erhält man 1 g (58 %) der Titelverbindung vom Schmp. 305-07°C.

## Patentansprüche

1. Verbindungen der allgemeinen Formel I 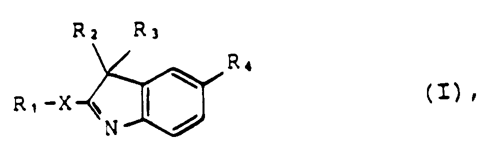 in welcher
R₁ einen Phenylring der allgemeinen Formel II darstellt, 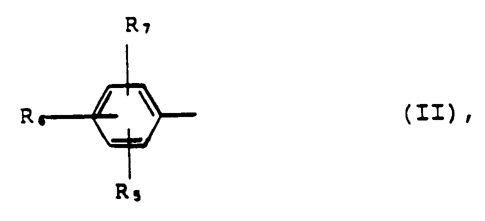 wobei R₅, R₆, R₇ gleich oder verschieden sein können und jeweils Wasserstoff, eine (C₁-C₅)Alkansulfonyloxy-, Trifluormethansulfonyloxy-, (C₁-C₅)Alkansulfonylamino-, Trifluormethansulfonylamino-, N-(C₁-C₅)Alkyl-(C₁-C₅)alkansulfonylamino-, N-(C₁-C₅)-Alkyltrifluormethansulfonyllamino-, (C₁-C₅)Alkylsulfenylmethyl-, (C₁-C₅)Alkylsulfinylmethyl- oder (C₁-C₅)Alkylsulfonylmethylgruppe, eine durch eine Hydroxy-, (C₁-C₅)Alkoxy-, Amino-, (C₁-C₅)Alkylamino- oder Di(C₁-C₅)alkylaminogruppe substituierte Carbonylgruppe, eine durch eine Amino-, (C₁-C₅)Alkylamino-, Di(C₁-C₅)alkylamino- oder cyclische Iminogruppe substituierte Sulfonylgruppe, wobei eine Methylengruppe in 4-Stellung durch ein Schwefel- oder Sauerstoffatom ersetzt sein kann, eine (C₁-C₅)Alkylcarbonylamino-, Aminocarbonylamino- oder (C₁-C₅)Alkylaminocarbonylaminogruppe, eine (C₁-C₅)Alkylmercapto-, (C₁-C₅)Alkylsulfinyl- oder (C₁-C₅)Alkylsulfonylgruppe, eine Nitro-, Halogen-, Amino-, Hydroxy-, (C₁-C₅)Alkyl-, (C₁-C₅)Alkoxy-, (C₁-C₅)Alkenyloxy-, (C₁-C₅)Alkinyloxy-, Cyan(C₁-C₅)alkyloxy-, Carboxy(C₁-C₅)alkyloxy-, (C₁-C₅)Alkoxycarbonyl-(C₁-C₅)alkyloxy-, Di(C₁-C₅)alkylamino-, 1-Imidazolyl-, Trifluormethyl- oder Cyangruppe sein können, oder
R₁ einen Naphthylrest oder einen gesättigten oder ungesättigten heterocyclischen Fünfring mit 1-4 Heteroatomen oder einen gesättigten oder ungesättigten heterocyclischen Sechsring mit 1-5 Heteroatomen darstellt, wobei die Heteroatome gleich oder verschieden sein können und Sauerstoff, Schwefel oder Stickstoff bedeuten und gewünschtenfalls an einem oder mehreren Stickstoffatomen ein Sauerstoffatom tragen können, und die Fünf- oder Sechsringe gegebenenfalls durch eine oder mehrere (C₁-C₆) Alkyl-, (C₁-C₆)Alkoxy-, (C₁-C₆)Alkylmercapto-, Hydroxy-, Nitro-, Amino-, Halogen- oder Cyangruppen substituiert oder mit einem Phenylring zu einem Bicyclus kondensiert sein können,
oder für den Fall, daß X eine Bindung darstellt, R₁ neben den oben genannten Gruppen auch ein Wasserstoffatom, eine (C₁-C₁₀)Alkyl-, (C₃-C₇)Cycloalkyl-, (C₂-C₁₀)Alkenyl-, (C₃-C₇)Cycloalkenyl-, (C₁-C₆)Alkoxy-(C₁-C₆)alkyl-, Carboxy(C₁-C₆)alkyl-, (C₁-C₆)Alkoxycarbonyl(C₁-C₆)alkyl-, Amino(C₁-C₆)alkyl-, Hydroxy(C₁-C₆)alkyl-, Amino-, (C₁-C₆)Alkylamino-, (C₁-C₆)Alkylcarbonylamino, Formylamino-, (C₁-C₆)Alkylsulfonylaminogruppe oder eine Alkinylgruppe mit bis zü 10 C-atomen bedeutet,
R₂ eine (C₁-C₆)Alkyl-, oder Phenylgruppe bedeutet,
R₃ eine (C₁-C₆)Alkyl-, oder Phenylgruppe bedeutet oder mit R₂ zusammen eine (C₃-C₇)Cycloalkylengruppe darstellt,
R₄ einen heterocyclischen Fünfring mit 1 - 4 Heteroatomen oder einen heterocyclischen Sechsring mit 1 - 5 Heteroatomen darstellt, wobei die Heteroatome der vorgenannten Fünf- und Sechsringe gleich oder verschieden sein können und Stickstoff, Sauerstoff oder Schwefel bedeuten und die vorgenannten Fünf- und Sechsringe gegebenenfalls durch eine oder mehrere (C₁-C₆)Alkyl-, (C₁-C₆)Alkoxy-, (C₁-C₆)Alkoxy(C₁-C₆)alkyl-, (C₁-C₆)Alkylmercapto-, Hydroxy-, Hydroxy(C₁-C₆)Alkyl-, Amino-, Halogen-, Oxo- oder Cyangruppen substituiert sein können,
X eine Bindung, eine (C₁-C₄)Alkylen-, die Vinylen-, die Iminogruppe -NH- oder die Carbonylaminogruppe -CONH-bedeutet,
deren Tautomere und deren physiologisch verträgliche Salze anorganischer oder organischer Säuren.

2. Verbindungen der Formel I gemäß Anspruch 1,
in der
R₁ einen Phenylring der Formel II darstellt, wobei R₅ Wasserstoff, eine (C₁-C₂)Alkylsulfonyloxy-, Trifluormethylsulfonyloxy-, (C₁-C₂)-Alkylsulfenylmethyl-, (C₁-C₂)-Alkylsulfinylmethyl-, (C₁-C₂)Alkylsulfonylmethyl-, (C₁-C₂)Alkylsulfonylamino-, N-(C₁-C₂)Alkyl(C₁-C₂)alkylsulfonylamino-, Trifluormethylsulfonylamino-, oder N-(C₁-C₂)Alkyl-trifluormethylsulfonylaminogruppe, eine durch eine Hydroxy-, (C₁-C₂)Alkoxy-, Amino-, (C₁-C₂)Alkylamino- oder Di-(C₁-C₂)Alkylaminogruppe substituierte Carbonylgruppe oder eine durch eine Amino-, Di(C₁-C₂)Alkylamino- oder Morpholinogruppe substituierte Sulfonylgruppe, eine Nitro-, Cyan- oder (C₁-C₄)Alkylaminosulfonylgruppe, eine (C₁-C₂)Alkylcarbonylamino-, Aminocarbonylamino- oder N-(C₁-C₂)Alkylaminocarbonylaminogruppe, eine (C₁-C₂)Alkylmercapto-, (C₁-C₂)Alkylsulfinyl- oder (C₁-C₂)Alkylsulfonylgruppe, eine Halogen-, Amino-, Hydroxy-, Di-(C₁-C₃)alkylamino-, (C₁-C₃)Alkyl-, (C₁-C₃)-Alkoxy-, (C₂-C₃)Alkenyloxy- oder (C₂-C₃)Alkinyloxygruppe, eine Cyanmethyloxy- oder Methoxycarbonylmethyloxygruppe, die Trifluormethylgruppe oder die 1-Imidazolylgruppe,
und
R₆ Wasserstoff, eine (C₁-C₃)Alkylgruppe, eine (C₁-C₂)-Alkoxy- oder Di(C₁-C₂)alkylaminogruppe oder ein Halogenatom und
R₇ Wasserstoff oder die Methoxygruppe,
oder R₁ einen Pyrrol-, Furan-, Thiophen-, Pyrazol-, Imidazol-, Thiazol-, Isothiazol-, Oxazol-, Isoxazol-, Triazol-, Tetrazol-, Thiadiazol-, Oxadiazol-, Pyrazin-, N,N-Dioxy-pyrazin-, Pyrimidin-, N,N-Dioxy-pyrimidin-, Pyridazin-, Oxazin-, Thiazin-, Triazin-, Tetrazin-, Pyridin-, N-Oxy-pyridin-, Piperidin-, Piperazin-, Morpholin- und Thiomorpholinrest,
oder für den Fall, daß X eine Bindung bedeutet, R₁ neben den oben genannten Gruppen auch ein Wasserstoffatom, eine (C₁-C₁₀)Alkyl-, (C₃-C₇)Cycloalkyl-, (C₂-C₁₀)Alkenyl-, (C₃-C₇)Cycloalkenyl-, (C₂-C₁₀)Alkinyl-, (C₁-C₆)Alkoxy-(C₁-C₆)-alkyl-, Carboxy(C₁-C₆)alkyl-, (C₁-C₆)Alkoxycarbonyl(C₁-C₆)alkyl-, Amino(C₁-C₆)alkyl-, Hydroxy(C₁-C₆)alkyl-, Amino-, (C₁-C₆)Alkylamino-, (C₁-C₆)Alkylcarbonylamino-, Formylamino- oder (C₁-C₆)Alkylsulfonylaminogruppe bedeutet,
R₂ eine gerade oder verzweigte (C₁-C₅)Alkylkette darstellt,
R₃ eine gerade oder verzweigte (C₁-C₅)Alkylkette, oder zusammen mit R₂ eine (C₃-C₇)Cycloalkylenkette darstellt,
R₄ einen 3-Oxo-2,3-dihydro-6-pyridazinyl-, 5-(C₁-C₆)Alkyl-3-oxo-2,3-dihydro-6-pyridazinyl-, 3-Oxo-2,3,4,5-tetrahydro-6-pyridazinyl-, 5-(C₁-C₆)Alkyl-3-oxo-2,3,4,5-tetrahydro-6-pyridazinyl-, 5-Hydroxy(C₁-C₆)alkyl-3-oxo-2,3,4,5-tetrahydro-6-pyridazinyl-, 3-Cyan-6-(C₁-C₆)alkyl-2-oxo-1,2-dihydro-5-pyridinyl-, 6-(C₁-C₆)-Alkyl-2-oxo-1,2-dihydro-5-pyridinyl-, 3-Amino-2-oxo-1,2-dihydro-5-pyridinyl-, 3-Amino-6-(C₁-C₆)alkyl-2-oxo-1,2-dihydro-5-pyridinyl-, 3-Hydroxy-6-(C₁-C₆)alkyl-2-oxo-1,2-dihydro-5-pyridinyl-, 3-Oxo-2H-3,4-dihydro-1,4-thiazin-6-yl-, 6-Oxo-1,6-dihydro-1,2,4-triazin-3-yl-, 6-Oxo-1,4,5,6-tetrahydro-1,2,4-triazin-3-yl-, 5-Oxo-4,5-dihydro-6H-1,3,4-oxadiazin-2-yl, 5-Oxo-4,5-dihydro-6H-1,3,4-thiadiazin-2-yl-, 3-Oxo-2,3-dihydro-1,2,4-triazin-6-yl-, 3-Oxo-2,3,4,5-tetrahydro-1,2,4-triazin-6-yl, 2-Oxo-2,3-dihydro-6H-1,3,4-oxadiazin-5-yl-, 2-Oxo-2,3-dihydro-6H-1,3,4-thiadiazin-5-yl-, 5-(C₁-C₆)Alkyl-3-oxo-2,3,4,5-tetrahydro-1,2,4-triazin-6-yl-, 2-Oxo-1,2-dihydro-5-pyrimidinyl-, 4-(C₁-C₆)Alkyl-2-oxo-1,2-dihydro-5-pyrimidinyl-, 2-Oxo-1,2-dihydro-5-pyrazinyl, 3-(C₁-C₆)Alkyl-2-oxo-1,2-dihydro-5-pyrazinyl-, 6-(C₁-C₆)-Alkyl-2-oxo-1,2-dihydro-5-pyrazinyl-, 4,4-Di(C₁-C₆)alkyl-5-oxo-4,5-dihydro-3-pyrazolyl-, 2-Oxo-4-pyrrolidinyl-, 3-(C₁-C₆)Alkyl-2-oxo-4-pyrrolidinyl-, 5-Oxo-4,5-dihydro-1,2,4-triazol-3-yl-, 4-(C₁-C₆)Alkyl-5-oxo-4,5-dihydro-1,2,4-triazol-3-yl-, 2-Oxo-2,3-dihydro-4(5)-imidazolyl- oder 5(4)-(C₁-C₆)Alkyl-2-oxo-2,3-dihydro-4(5)-imidazolylrest bedeutet,
X eine Bindung, eine (C₁-C₂)Alkylen-, die Vinylen-, die Iminogruppe -NH- oder die Carbonylaminogruppe -CONH-bedeutet.

3. Verbindungen der allgemeinen Formel I gemäß Anspruch 1 oder 2, in der
R₁ den Phenylrest der allgemeinen Formel II bedeutet, in der R₅ ein Wasserstoffatom, die Methansulfonyloxy-, Trifluormethansulfonyloxy-, Methansulfonylamino-, Trifluormethansulfonylamino-, Methylmercapto-, Methylsulfinyl-, Methylsulfonyl-, Hydroxy-, Methyl-, Metoxy-, Propargyloxy-, Trifluormethyl- oder die 1-Imidazolylgruppe, R₆ Wasserstoff, die Methyl-, Methoxy-, Dimethylamino- oder Chlorgruppe und R₇ Wasserstoff oder die Methoxygruppe bedeutet, oder
R₁ den Pyrrol-, Furan-, Thiophen-, Pyrazol-, Imidazol-, Isothiazol-, Thiazol-, Oxazol-, Triazol-, Tetrazol-, Thiadiazol-, Isoxazol-, Oxadiazol-, Pyridin-, N-Oxypyridin-, Pyrazin-, N,N-Dioxypyrazin, Pyrimidin-, N,N-Dioxypyrimidin-, Pyridazin-, Oxazin-, Thiazin-, Triazin-, Tetrazin-, Piperidin-, Piperazin-, Morpholin- oder Thiomorpholinrest darstellt sowie deren Methyl-, Ethyl-, Methoxy-, Ethoxy-, Methylmercapto-, Ethylmercapto- und Chlor- substituierten Derivate, oder R₁ den Indol-, Indazol-, Chinolin-, Isochinolinrest oder Naphthylrest darstellt, oder
R₁ für den Fall, daß X eine Bindung darstellt, neben den genannten Gruppen auch ein Wasserstoffatom, die Methyl-, Ethyl-, Propyl-, Butyl-, Pentyl-, Hexyl-, Propenyl-, Cyclopentyl-, Cyclohexyl-, Methylamino-, Amino-, Acetamido- oder Formamidogruppe bedeutet,
R₂ und R₃ gleich sind und Methylgruppen bedeuten oder R₂ und R₃ zusammen mit dem Kohlenstoffatom, an das sie gebunden sind, einen Cyclopentanring oder Cyclohexanring bilden,
R₄ den 3-Oxo-2-,3,4,5-tetrahydro-6-pyridazinyl-, 5-Methyl-3-oxo-2,3,4,5-tetrahydro-6-pyridazinyl-, 5-Hydroxymethyl-3-oxo-2,3,4,5-tetrahydro-6-pyridazinyl-, 3-Oxo-2,3-dihydro-6-pyridazinyl-, 3-Cyan-6-methyl-2-oxo-1,2-dihydro-5-pyridinyl-, 3-oxo-2,3,4,5-tetrahydro-1,2,4-triazin-6-yl, 2-Oxo-4,5-dihydro-6H-1,3,4-oxadiazin-5-yl-, 6-Oxo-1,4,5,6-tetrahydro-1,2,4-triazin-3-yl-, 5-Oxo-4,5-dihydro-6H-1,3,4-oxadiazin-2-yl-, 2-Oxo-1,2-dihydro-5-pyrimidinyl-, 2-Oxo-1,2-dihydro-5-pyrazinyl-, 4,4-Dimethyl-5-oxo-4,5-dihydro-3-pyrazolyl-, 2-Oxo-4-pyrrolidinyl-, 4-Methyl-5-oxo-4,5-dihydro-1,2,4-triazol-3-yl- oder den 5(4)-Methyl-2-oxo-2,3-dihydro-4(5)-imidazolyrest und
X einen Valenzstrich, die Vinylengruppe, die Methylengruppe, die Iminogruppe oder Carbonylaminogruppe bedeutet.

4. Verbindung der allgemeinen Formel I gemäß Anspruch 1, 2 oder 3, in der
R₁ einen unsubstituierten oder durch (C₁-C₅)Alkyl-, (C₁-C₅)Alkoxy-, Halogen- oder (C₁-C₅)Alkylmercaptogruppen substituierten Phenylring, oder eine Pyridyl- oder Thienylgruppe, oder für den Fall, daß X eine Bindung oder die Iminogruppe -NH- bedeutet, R1 auch eine (C₁-C₆)Alkylgruppe darstellt,
R₂ und R₃ gleich sind und Methylgruppen bedeuten, oder R₂ und R₃ zusammen mit dem Kohlenstoffatom, an das sie gebunden sind, einen Cyclopentan- oder Cyclohexanring bilden,
R₄ den 3-Oxo-2,3,4,5-tetrahydro-6-pyridazinyl-, 5-Methyl-3-oxo-2,3,4,5-tetrahydro-6-pyridazinyl- oder 3-Oxo-2,3-dihydro-6-pyridazinylrest bedeutet, und
X eine Bindung darstellt.

5. Verfahren zur Herstellung von Verbindungen der Formel I gemäß den Ansprüchen 1-4, dadurch gekennzeichnet, daß man in an sich bekannter Weise
A) Verbindungen der allgemeinen Formel VI 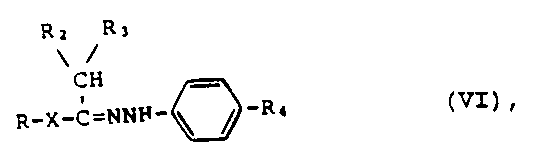 in der R₁, R₂, R₃, R₄ und X die in den Ansprüchen 1 - 4 angegebenen Bedeutungen haben, durch eine Fischer-Indol-Synthese cyclisiert,
oder
B) im Falle, daß R₄ einen gegebenenfalls ein- oder mehrfach durch (C₁-C₆)Alkyl-, (C₁-C₆)Alkoxy-, Hydroxy- oder Oxogruppen substituierten Pyridazinylrest darstellt, Verbindungen der allgemeinen Formel XI 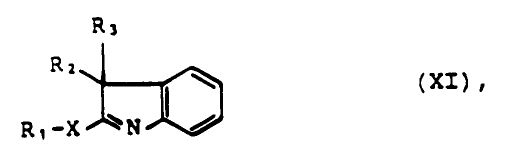 in der R₁, R₂, R₃, R₄ und X die in den Ansprüchen 1 - 4 angegebenen Bedeutungen haben, mit Säurechloriden der allgemeinen Formel XII
R₈OOC-CH₂-CHR₉-COCl (XII),
in der R₈ einen (C₁-C₆)Alkylrest und R₉ Wasserstoff oder eine (C₁-C₆)Alkylgruppe ist, oder mit Carbonsäureanhydriden der allgemeinen Formel XIII 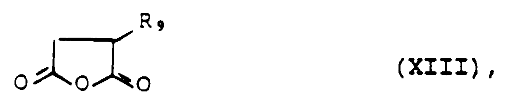 in der R₉ die oben genannte Bedeutung hat, zu den Verbindungen der allgemeinen Formel XIV oder deren R₈-Ester 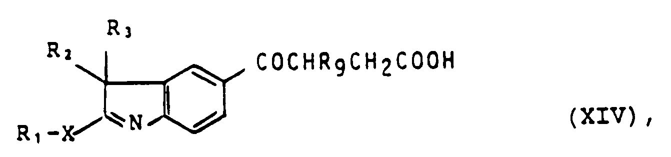 in der R₁, R₂, R₃, R₉ und X die oben angegebenen Bedeutungen haben, umsetzt, und die so erhaltenen Verbindungen mit Hydrazin oder einem geeigneten Hydrazin-Derivat zu Verbindungen der allgemeinen Formel I, in der R₄ einen gegebenenfalls durch (C₁-C₆)Alkyl-, (C₁-C₆)Alkoxy-, Hydroxy- oder Oxogruppen substituierten Pyridazinylrest darstellt, cyclisiert,
und anschließend die so erhaltenen Verbindungen der allgemeinen Formel I gewünschtenfalls in andere Verbindungen der allgemeinen Formel I nachträglich umwandelt, sowie diese Verbindungen gegebenenfalls in ihre pharmakologisch verträglichen Salze überführt.

6. Arzneimittel enthaltend mindestens eine Verbindung der Formel I gemäß einem der Ansprüche 1 - 4 sowie gegebenenfalls pharmakologisch übliche Träger- und Hilfsstoffe.

7. Verwendung von Verbindungen der Formel I gemäß einem der Ansprüche 1 - 4 zur Herstellung von Arzneimitteln mit positiv inotroper Wirkung.

8. Verfahren zur Herstellung von Arzneimitteln enthaltend mindestens eine Verbindung der Formel I gemäß einem der Ansprüche 1 - 4, dadurch gekennzeichnet, daß man eine Verbindung der Formel I mit pharmakologisch üblichen Träger- und Hilfsstoffen vermischt.

9. Verbindungen der Formel VI 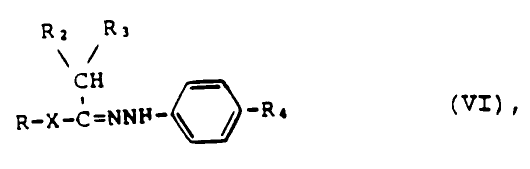 in welcher
R₁ einen Phenylring der allgemeinen Formel II darstellt, 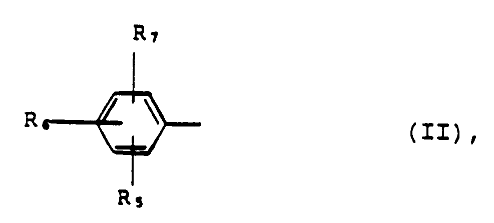 wobei R₅, R₆, R₇ gleich oder verschieden sein können und jeweils Wasserstoff, eine (C₁-C₅)Alkansulfonyloxy-, Trifluormethansulfonyloxy-, (C₁-C₅)Alkansulfonylamino-, Trifluormethansulfonylamino-, N-(C₁-C₅)Alkyl-(C₁-C₅)alkansulfonylamino-, N-(C₁-C₅)-Alkyltrifluormethansulfonylamino-, (C₁-C₅)Alkylsulfenylmethyl-, (C₁-C₅)Alkylsulfinylmethyl- oder (C₁-C₅)Alkylsulfonylmethylgruppe, eine durch eine Hydroxy-, (C₁-C₅)Alkoxy-, Amino-, (C₁-C₅)Alkylamino- oder Di(C₁-C₅)alkylaminogruppe substituierte Carbonylgruppe, eine durch eine Amino-, (C₁-C₅)Alkylamino-, Di(C₁-C₅)alkylamino- oder cyclische Iminogruppe substituierte Sulfonylgruppe, wobei eine Methylengruppe in 4-Stellung durch ein Schwefel- oder Sauerstoffatom ersetzt sein kann, eine (C₁-C₅)Alkylcarbonylamino-, Aminocarbonylamino- oder (C₁-C₅)Alkylaminocarbonylaminogruppe, eine (C₁-C₅)Alkylmercapto-, (C₁-C₅)Alkylsulfinyl- oder (C₁-C₅)Alkylsulfonylgruppe, eine Nitro-, Halogen-, Amino-, Hydroxy-, (C₁-C₅)Alkyl-, (C₁-C₅)Alkoxy-, (C₁-C₅)Alkenyloxy-, (C₁-C₅)Alkinyloxy-, Cyan(C₁-C₅)alkyloxy-, Carboxy(C₁-C₅)alkyloxy-, (C₁-C₅)Alkoxycarbonyl-(C₁-C₅)alkyloxy-, Di(C₁-C₅)alkylamino-, 1-Imidazolyl-, Trifluormethyl- oder Cyangruppe sein können, oder
R₁ einen Naphthylrest bedeutet oder R₁ einen gesättigten oder ungesättigten heterocyclischen Fünfring mit 1-4 Heteroatomen oder einen gesättigten oder ungesättigten heterocyclischen Sechsring mit 1-5 Heteroatomen darstellt, wobei die Heteroatome gleich oder verschieden sein können und Sauerstoff, Schwefel oder Stickstoff bedeuten und gewünschtenfalls an einem oder mehreren Stickstoffatomen ein Sauerstoffatom tragen können, und die Fünf- oder Sechsringe gegebenenfalls durch eine oder mehrere (C₁-C₆)Alkyl-, (C₁-C₆)Alkoxy-, (C₁-C₆)Alkylmercapto-, Hydroxy-, Nitro-, Amino-, Halogen- oder Cyangruppen substituiert oder mit einem Phenylring zu einem Bicyclus kondensiert sein können,
oder für den Fall, daß X eine Bindung darstellt, R₁ neben den oben genannten Gruppen auch ein Wasserstoffatom, eine (C₁-C₁₀)Alkyl-, (C₃-C₇)Cycloalkyl-, (C₂-C₁₀)Alkenyl-, (C₃-C₇)Cycloalkenyl-, (C₁-C₆)Alkoxy(C₁-C₆)alkyl-, Carboxy(C₁-C₆)alkyl-, (C₁-C₆)Alkoxycarbonyl(C₁-C₆)alkyl-, Amino(C₁-C₆)alkyl-, Hydroxy(C₁-C₆)alkyl-, Amino-, (C₁-C₆)Alkylamino-, (C₁-C₆)Alkylcarbonylamino, Formylamino-, (C₁-C₆)Alkylsulfonylaminogruppe oder eine Alkinylgruppe mit bis zu 10 C-atomen bedeutet,
R₂ eine (C₁-C₆)Alkyl-, oder Phenylgruppe bedeutet,
R₃ eine (C₁-C₆)Alkyl-, oder Phenylgruppe bedeutet oder mit R₂ zusammen eine (C₃-C₇)Cycloalkylengruppe darstellt,
R₄ einen heterocyclischen Fünfring mit 1 - 4 Heteroatomen oder einen heterocyclischen Sechsring mit 1 - 5 Heteroatomen darstellt, wobei die Heteroatome der vorgenannten Fünf- und Sechsringe gleich oder verschieden sein können und Stickstoff, Sauerstoff oder Schwefel bedeuten und die vorgenannten Fünf- und Sechsringe gegebenenfalls durch eine oder mehrere (C₁-C₆)Alkyl-, (C₁-C₆)Alkoxy-, (C₁-C₆)Alkoxy(C₁-C₆)Alkyl-, (C₁-C₆)Alkylmercapto-, Hydroxy-, Hydroxy(C₁-C₆)alkyl-, Amino-, Halogen-, Oxo- oder Cyangruppen substituiert sein können,
X eine Bindung, eine (C₁-C₄)Alkylen-, die Vinylen-, die Iminogruppe -NH- oder die Carbonylaminogruppe -CONH-bedeutet,
mit Ausnahme der Verbindung 6-(4-sec.Butylidenhydrazinophenyl)-5-methyl-4,5-dihydro-1,2,4-triazin-3(2H)-on.

## Claims

1. Compounds of the general formula I 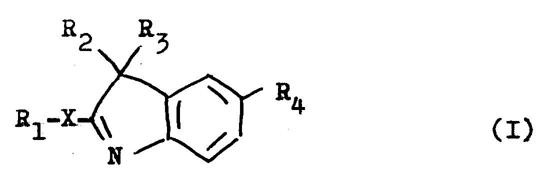 in which R₁ represents a phenyl ring of the general formula II 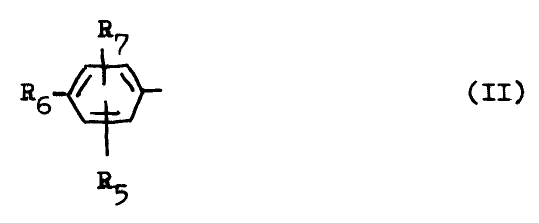 whereby R₅, R₆, R₇ can be the same or different and in each case can be hydrogen, a (C₁-C₅)-alkanesulphonyloxy, trifluoromethanesulphonyloxy, (C₁-C₅)-alkenesulphonylamino, trifluoromethanesulphonylamino, N-(C₁-C₅)alkyl-(C₁-C₅)-alkanesulphonylamino, N-(C₁-C₅)-alkyl-trifluoromethanesulphonylamino, (C₁-C₅)-alkylsulphenylmethyl, (C₁-C₅)alkylsulphinylmethyl or (C₁-C₅)alkylsulphonylmethyl group, a carbonyl group substituted by a hydroxyl, (C₁-C₅)-alkoxy, amino, (C₁-C₅)-alkylamino or di-(C₁-C₅)-alkylamino group, a sulphonyl group substituted by amino, (C₁-C₅)-alkylamino, di-(C₁-C₅)-alkylamino or cyclic imino group, whereby a methylene group in the 4-position can be replaced by a sulphur or oxygen atom, a (C₁-C₅)-alkylcarbonylamimo, aminocarbonylamino or (C₁-C₅)-alkylaminocarbonylamino group, a (C₁-C₅)-alkylmercapto, (C₁-C₅)-alkylsulphinyl or (C₁-C₅)-alkylsulphonyl group, a nitro, halogen, amino, hydroxyl, (C₁-C₅)-alkyl, (C₁-C₅)-alkoxy, (C₁-C₅)alkenyloxy, (C₁-C₅)alkynyloxy, cyano-(C₁-C₅)-alkyloxy, carboxy-(C₁-C₅)-alkyloxy, (C₁-C₅)-alkoxycarbonyl-(C₁-C₅)alkyloxy, di-(C₁-C₅)-alkylamino, 1-imidazolyl, trifluoromethyl or cyane group or R₁ represents a naphthyl radical or a saturated or unsaturated heterocyclic five-membered ring with 1 - 4 heteroatoms or a saturated or unsaturated heterocyclic six-membered ring with 1 - 5 heteroatoms, whereby the heteroatoms can be the same or different and signify oxygen, sulphur or nitrogen and, if desired, can carry an oxygen atom on one or more nitrogen atoms and the five- and six-membered rings can possibly be substituted by one or more (C₁-C₆)-alkyl (C₁-C₆)-alkoxy, (C₁-C₆)-alkylmercapto, hydroxyl, nitro, amino, halogen or cyano groups or can be condensed with a phenyl ring to give a bicycle or, for the case that X represents a bond, besides the above-mentioned groups, R₁ also signifies a hydrogen atom, a (C₁-C₁₀)-alkyl, (C₃-C₇)-cycloalkyl, (C₂-C₁₀)-alkenyl, (C₃-C₇)-cycloalkenyl, (C₁-C₆)-alkoxy-(C₁-C₆)alkyl, carboxy-(C₁₋C₆)-alkyl, (C₁-C₆)alkoxycarbonyl-(C₁-C₆)-alkyl, amino-(C₁-C₆)-alkyl, hydroxy-(C₁-C₆)alkyl, amino, (C₁-C₆)-alkylamino, (C₁-C₆)-alkylcarbonylamino, formylamino, (C₁-C₆)-alkylsulphonylamino group or an alkynyl group with up to 10 C-atoms, R₂ signifies a (C₁-C₆)-alkyl or phenyl group, R₃ signifies a (C₁-C₆)-alkyl or phenyl group or, together with R₂, represents a (C₃-C₇)-cycloalkylene group, R₄ represents a heterocyclic five-membered ring with 1 - 4 heteroeatoms or a heterocyclic six-membered ring with 1 - 5 heteroatoms, whereby the heteroatoms of the above-mentioned five- and six-membered rings can be the same or different and signify nitrogen, oxygen or sulphur and the above-mentioned five- and six-membered rings can possibly be substituted by one or more (C₁-C₆)-alkyl, (C₁-C₆)-alkoxy, (C₁-C₆)-alkoxy-(C₁-C₆)-alkyl, (C₁-C₆)-alkylmercapto, hydroxyl, hydroxy-(C₁-C₆)-alkyl, amino, halogen, oxo or cyano groups, X signifies a bond, a (C₁-C₄)-alkylene, the vinylane, the imino group -NH- or the carbonylamino group -CONH-, their tautomers and their physiologically compatible salts of inorganic or organic acids.

2. Compounds of the formula I according to claim 1, in which R₁ represents a phenyl ring of the formula II, whereby R₅ signifies hydrogen, a (C₁-C₂)-alkylsulphonyloxy, trifluoromethylsulphonyloxy, (C₁-C₂)-alkylsulphenylmethyl, (C₁-C₂)alkylsulphinylmethyl, (C₁-C₂)-alkylsulphonylmethyl, (C₁-C₂)-alkylsulphonylamino, N-(C₁-C₂)-alkyl-(C₁-C₂)-alkylsulphonylamino, trifluoromethylsulphonylamino or N-(C₁-C₂)-alkyltrifluoromethylsulphonylamino group, a carbonyl group substituted by a hydroxyl, (C₁-C₂)alkoxy, amino, (C₁-C₂)-alkylamino or di-(C₁-C₂)-alkylamino group or a sulphonyl group substituted by an amino, di-(C₁-C₂)alkylamino or morpholino group, a nitro, cyano or (C₁-C₄)alkylaminosulphonyl group, a (C₁-C₂)-alkylcarbonylamino, aminocarbonylamino or N-(C₁-C₂)-alkylaminocarbonylamino group, a (C₁-C₂)-alkylmercapto, (C₁-C₂)-alkylsulphinyl or (C₁-C₂)-alkylsulphonyl group, a halogen, amino, hydroxyl, di-(C₁-C₃)-alkylamino, (C₁-C₃)-alkyl, (C₁-C₃)-alkoxy, (C₂-C₃)-alkenyloxy or (C₂-C₃)alkynyloxy group, a cyanomethyloxy or methoxycarbonylmethyloxy group, the trifluoromethyl group or the 1-imidazolyl group and R₆ hydrogen, a (C₁-C₃)-alkyl group, a (C₁-C₂)-alkoxy or di-(C₁-C₂)alkylamino group or a halogen atom and R₇ hydrogen or the methoxy group or R₁ a pyrrole, furan, thiophene, pyrazole, imidazole, thiazole, isothiazole, oxazole, isoxazole, triazole-, tetrazole, thiadiazole, oxadiazole, pyrazine, N,N-dioxypyrazine, pyrimidine, N,N-dioxypyrimidine, pyridazine, oxazine, thiazine, triazine, tetrazine, pyridine, N-oxypyridine, piperidine, piperazine, morpholine and thiomorpholine radical or, for the case that X signifies a bond, besides the above-mentioned groups, R₁ also signifies a hydrogen atom, a (C₁-C₁₀)alkyl, (C₃-C₇)-cycloalkyl, (C₂-C₁₀)alkenyl, (C₃-C₇)-cycloalkenyl, (C₂-C₁₀) alkynyl, (C₁-C₆)alkoxy-(C₁-C₆)-alkyl, carboxy-(C₁-C₆)-alkyl, (C₁-C₆)-alkoxycarbonyl-(C₁-C₆)-alkyl,- amino-(C₁-C₆)-alkyl, hydroxy-(C₁-C₆)-alkyl-, amino, (C₁-C₆)-alkylamino-, (C₁-C₆)alkylcarbonylamino, formylamino or (C₁-C₆)-alkylsulphonylamino group, R₂ represents a stright or branched (C₁-C₅)-alkyl chain, R₃ represents a straight or branched (C₁-C₅)alkyl chain or, together with R₂, a (C₃-C₇)-cycloalkylene chain, R₄ signifies a 3-oxo-2,3-dihydro-6-pyridazinyl, 5-(C₁-C₆)-alkyl-3-oxo-2,3-dihydro-6-pyridazinyl, 3-oxo-2,3,4,5-tetrahydro-6-pyridazinyl, 5-(C₁-C₆)-alkyl-3-oxo-2,3,4,5-tetrahydro-6-pyridazinyl, 5-hydroxy-(C₁-C₆)-alkyl-3-oxo-2,3,4,5-tetrahydro-6-pyridazinyl, 3-cyano-6-(C₁-C₆)-alkyl-2-oxo-1,2-dihydro-5-pyridinyl, 6-(C₁-C₆)-alkyl-2-oxo-1,2-dihydro-5-pyridinyl, 3-amino-2-oxo-1,2-dihydro-5-pyridinyl, 3-amino-6-(C₁-C₆)alkyl-2-oxo-1,2-dihydro-5-pyridinyl, 3-hydroxy-6-(C₁-C₆)-alkyl-2-oxo-1,2-dihydro-5-pyridinyl, 3-oxo-2H-3,4-dihydro-1,4-thiazin-6-yl, 6-oxo-1,6-dihydro-1,2,4-triazin-3-yl, 6-oxo-1,4,5,6-tetrahydro-1,2,4-triazin-3-yl, 5-oxo-4,5-dihydro-6H-1,3,4-oxadiazin-2-yl, 5-oxo-4,5-dihydro-6H-1,3,4-thiadiazin-2-yl, 3-oxo-2,3-dihydro-1,2,4-triazin-6-yl, 3-oxo-2,3,4,5-tetrahydro-1,2,4-triazin-6-yl, 2-oxo-2,3-dihydro-6H-1,3,4-oxadiazin-5-yl, 2-oxo-2,3-dihydro-6H-1,3,4-thiadiazin-5-yl, 5-(C₁-C₆)-alkyl-3-oxo-2,3,4,5-tetrahydro-1,2,4-triazin-6-yl, 2-oxo-1,2-dihydro-5-pyrimidinyl, 4-(C₁-C₆)-alkyl-2-oxo-1,2-dihydro-5-pyrimidinyl, 2-oxo-1,2-dihydro-5-pyrazinyl, 3-(C₁-C₆)-alkyl-2-oxo-1,2-dihydro-5-pyrazinyl, 6-(C₁-C₆)-alkyl-2-oxo-1,2-dihydro-5-pyrazinyl, 4,4-di-(C₁-C₆)-alkyl-5-oxo-4,5-dihydro-3-pyrazolyl, 2-oxo-4-pyrrolidinyl, 3-(C₁-C₆)-alkyl-2-oxo-4-pyrrolidinyl, 5-oxo-4,5-dihydro-1,2,4-triazol-3-yl, 4-(C₁-C₆)-alkyl-5-oxo-4,5-dihydro-1,2,4-triazol-3-yl, 2-oxo-2,3-dihydro-4(5)-imidazolyl or 5(4)-(C₁-C₆)-alkyl-2-oxo-2,3-dihydro-4(5)-imidazolyl radical, X signifies a bond, a (C₁-C₂)-alkylene, the vinylene, the imino group -NH- or the carbonylamino group -CONH-.

3. Compounds of the general formula I according to claim 1 or 2, in which R₁ signifies a phenyl radical of the general formula II in which R₅ signifies a hydrogen atom, the methanesulphonyloxy, trifluoromethanesulphonyloxy, methanesulphonylamino, trifluoromethanesulphonylamino, methylmercapto, methylsulphinyl, methylsulphonyl, hydroxyl, methyl, methoxy, propargyloxy, trifluoromethyl or the 1-imidazolyl group, R₆ signifies hydrogen, the methyl, methoxy, dimethylamino or chlorine group and R₇ signifies hydrogen or the methoxy group or R₁ represents the pyrrole, furan, thiophene, pyrazole, imidazole, isothiazole, thiazole, oxazole, triazole, tetrazole, thiadiazole, isoxazole, oxadiazole, pyridine, N-oxypyridine, pyrazine, N,N-dioxypyrazine, pyrimidine, N,N-dioxypyrimidine, pyridazine, oxazine, thiazine, triazine, tetrazine, piperidine, piperazine, morpholine or thiomorpholine radical, as wells as their methyl-, ethyl-, methoxy-, ethoxy-, methylmercapto-, ethylmercapto- and chlorine-substituted derivatives, or R₁ represents the indole, indazole, quinoline, isoquinoline radical or naphthyl radical or, for the ease that X represents a bond, besides the above-mentioned groups, R₁ also signifies a hydrogen atom, the methyl, ethyl, propyl, butyl, pentyl, hexyl, propenyl, cyolopentyl, cyclohexyl, methylamine, amino, acetamido or formamide groups, R₂ and R₃ are the same and signify methyl groups or R₂ and R₃, together with the carbon atom to which they are attached, form a cyclopentane ring or cyclohexane ring, R₄ signifies the 3-oxo-2,3,4,5-tetrahydro-6-pyridazinyl, 5-methyl-3-oxo-2,3,4,5-tetrahydro-6-pyridazinyl, 5-hydroxymethyl-3-oxo-2,3,4,5-tetrahydro-6-pyridazinyl, 3-oxo-2,3-dihydro-6-pyridazinyl, 3-cyano-6-methyl-2-oxo-1,2-dihydro-5-pyridinyl, 3-oxo-2,3,4,5-tetrahydro-1,2,4-triazin-6-yl, 2-oxo-4,5-dihydro-6H-1,3,4-oxadiazin-5-yl, 6-oxo-1,4,5,6-tetrahydro-1,2,4-triazin-3-yl, 5-oxo-4,5-dihydro-6H-1,3,4-oxadiazin-2-yl, 2-oxo-1,2-dihydro-5-pyrimidinyl, 2-oxo-1,2-dihydro-5-pyrazinyl, 4,4-dimethyl-5-oxo-4,5-dihydro-3-pyrazolyl, 2-oxo-4-pyrrolidinyl, 4-methyl-5-oxo-4,5-dihydro-1,2,4-triazol-3-yl or the 5(4)-methyl-2-oxo-2,3-dihydro-4(5)-imidazolyl radical and X represents a valency bond, the vinylene group, the methylene group, the imino group or carbonylamino groups.

4. Compounds of the general formula I according to claim 1, 2 or 3, in which R₁ represents a phenyl ring unsubstituted or substituted by (C₁-C₅)alkyl, (C₁-C₅)-alkoxy, halogen or (C₁-C₅)alkylmercapto or is a pyridyl or thienyl group or, for the case that X represents a bond or the imino groups -NH-, R₁ also represents a (C₁-C₆)-alkyl group, R₂ and R₃ are the same and signify methyl groups or R₂ and R₃, together with the carbon atom to which they are attached, form a cyclopentane or cyclohexane ring, R₄ signifies the 3-oxo-2,3,4,5-tetrahydro-6-pyridazinyl, 5-methyl-3-oxo-2,3,4,5-tetrahydro-6-pyridazinyl or 3-oxo-2,3-dihydro-6-pyridazinyl radical and X represents a bond.

5. Process for the preparation of compounds of the formula I according to claims 1 - 4, characterised in that, in per se known manner, one
A) cyclises compounds of the general formula VI 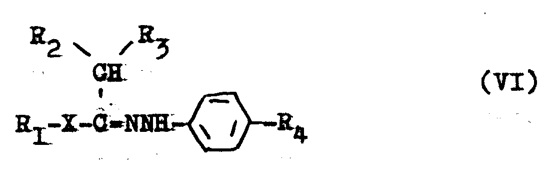 in which R₁, R₂, R₃, R₄ and X have the meanings given in claims 1 - 4, by a Fischer indole synthesis; or
B) in the case that R₄ represents a pyridazinyl radical possibly substituted one or more times by (C₁-C₆)alkyl, (C₁-C₆)-alkoxy, hydroxyl or oxo groups, reacts compounds of the general formula XI 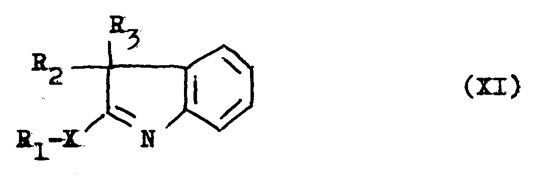 in which R₁, R₂, R₃, R₄ and X have the meanings given in claims 1 - 4, with acid chlorides of the general formula XII
R₈OOC-CH₂-CHR₉-COCl (XII)
in which R₈ is a (C₁-C₆)alkyl radical and R₉ hydrogen or a (C₁-C₆)-alkyl group, or with carboxylic acid anhydrides of the general formula XIII 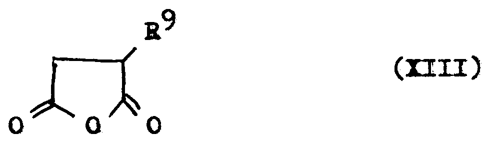 in which R₉ has the above-given meaning, to give the compounds of the general formula XIV or their R₈ esters 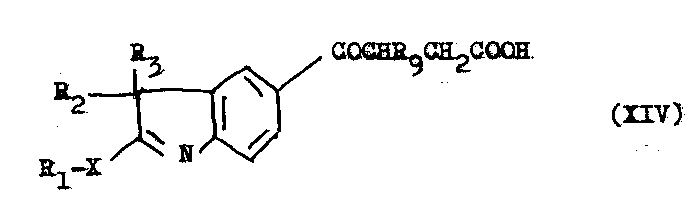 in which R₁, R₂, R₃, R₉ and X have the above-given meanings, and cyclises the so-obtained compounds with hydrazine or a suitable hydrazine derivative to give compounds of the general formula I, in which R₄ represents a pyridazinyl radical possibly substituted by (C₁-C₆)-alkyl, (C₁-C₆)-alkoxy, hydroxyl or oxo, and subsequently, if desired, converts the so-obtained compounds of the general formula I into other compounds of the general formula I, as well as possibly converts these compounds into their pharmacologically compatible salts.

6. Medicaments containing at least one compound of the formula I according to one of claims 1 - 4, as well as possibly pharmacologically usual carrier and adjuvant materials.

7. Use of compounds of the formula I according to one of claims 1 - 4 for the preparation of medicaments with positive inotropic action.

8. Process for the preparation of medicaments containing at least one compound of the formula I according to one of claims 1 - 4, characterised in that one mixes a compound of the formula I with pharmacologically usual carrier and adjuvant materials,

9. Compounds of the general formula VI 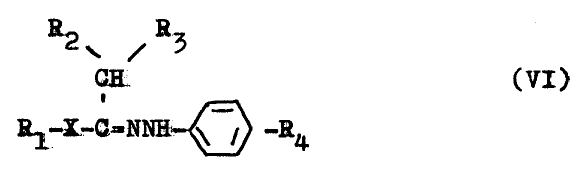 in which R₁ represents a phenyl ring of the general formula II 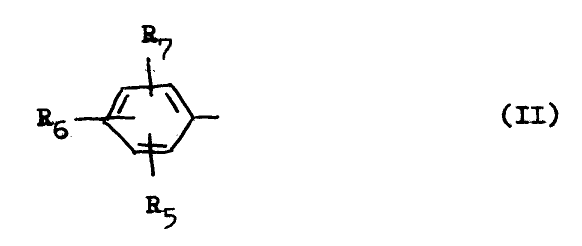 whereby R₅, R₆, R₇ can be the same or different and in each case can be hydrogen, a (C₁-C₅)alkanesulphonyloxy, trifluoromethanesulphonyloxy, (C₁-C₅)alkanesulphonylamino, trifluoromethanesulphonylamino, N-(C₁-C₅)alkyl-(C₁-C₅)-alkanesulphonylamino, N-(C₁-C₅)-alkyltrifluoromethanesulphonylamino, (C₁-C₅)-alkylsulphenylmethyl, (C₁-C₅)alkylsulphinylmethyl or (C₁-C₅)alkylsulphonylmethyl group, a carbonyl group substituted by a hydroxyl, (C₁-C₅)-alkoxy, amino, (C₁-C₅)-alkylamino or di-(C₁-C₅)-alkylamino group, a sulphonyl group substituted by an amino, (C₁-C₅)-alkylamino, di-(C₁-C₅)-alkylamino or cyclic imino group, whereby a methylene group in 4-position can be replaced by a sulphur or oxygen atom, a (C₁-C₅)-alkylcarbonylamino, aminocarbonylamino or (C₁-C₅)-alkylaminocarbonylamino group, a (C₁-C₅)-alkylmercapto, (C₁-C₅)-alkylsulphinyl or (C₁-C₅)-alkylsulphonyl group, a nitro, halogen, amino, hydroxyl, (C₁-C₅)-alkyl, (C₁-C₅)-alkoxy, (C₁-C₅)-alkenyloxy, (C₁-C₅)alkynyloxy, cyano-(C₁-C₅)-alkyloxy, carboxy-(C₁-C₅)alkyloxy, (C₁-C₅)-alkoxycarbonyl-(C₁-C₅) alkyloxy, di-(C₁-C₅)-alkylamino, 1-imidazolyl, trifluoromethyl or cyano group, or R₁ signifies a naphthyl radical or R₁ represents a saturated or unsaturated heterocyclic five-membered ring with 1 - 4 heteroatoms or a saturated or unsaturated heteracyolic six-membered ring with 1 - 5 heteroatoms, whereby the heteroatoms can be the same or different and signify oxygen, sulphur or nitrogen and, if desired, can carry an oxygen atom on one or more nitrogen atoms, and five- or six-membered rings can possibly be substituted by one or more (C₁-C₆)-alkyl, (C₁-C₆)-alkoxy, (C₁-C₆)-alkylmercapto, hydroxyl, nitro, amino, halogen or cyano groups or can be condensed with a phenyl ring to give a bicycle, or for the case that X represents a bond, besides the above-mentioned groups, R₁ also signifies a hydrogen atom, a (C₁-C₁₀)-alkyl, (C₃-C₇)-cycloalkyl, (C₂-C₁₀)-alkenyl, (C₃-C₇)-cycloalkenyl, (C₁-C₆)-alkoxy-(C₁-C₆)-alkyl, carboxy-(C₁-C₆)alkyl, (C₁-C₆)-alkoxycarbonyl-(C₁-C₆)-alkyl, amino-(C₁-C₆)-alkyl, hydroxy-(C₁-C₆)-alkyl, amino, (C₁-C₆)-alkylamino, (C₁-C₆)-alkylcarbonylamino, formylamino, (C₁-C₆)-alkylsulphonylamino group or an alkynyl group with up to 10 C-atoms, R₂ signifies a (C₁-C₆)-alkyl or phenyl group, R₃ represents a (C₁-C₆)-alkyl or phenyl group or, together with R₂, represents a (C₃-C₇)-cycloalkylene group, R₄ represents a heterocyclic five-membered ring with 1 - 4 heteroatoms or a heterocyclic six-membered ring with 1 - 5 heteroatoms, whereby the heteroatoms of the above-mentioned five- and six-membered rings can be the same or different and signify nitrogen, oxygen or sulphur and the above-mentioned five- and six-membered rings can possibly be substituted by one or more (C₁-C₆)-alkyl, (C₁-C₆)-alkoxy, (C₁-C₆)-alkoxy-(C₁-C₆)-alkyl, (C₁-C₆)-alkylmercapto, hydroxyl, hydroxy-(C₁-C₆)-alkyl, amino, halogen, oxo or cyano groups, X signifies a bend, a (C₁-C₄)-alkylene, the vinylene, the imino group -NH) or the carbonylamine group -CONH-, with the exception of the compound 6-(4-sec,-butylidenehydrazinophenyl)-5-methyl-4,5-dihydro-1,2,4-triazin-3(2H)-one.

## Revendications

1. Composés de formule générale I 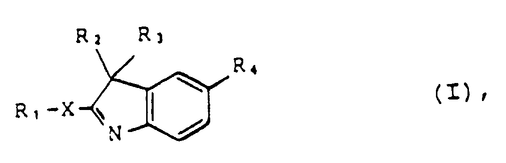 dans laquelle
R₁ représente un noyau phényle de formule générale II 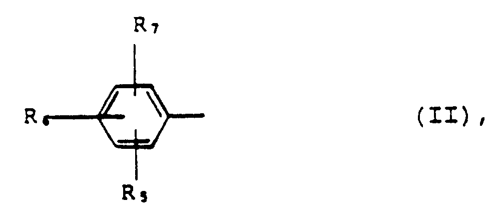 dans laquelle
R₅, R₆, R₇ peuvent être identiques ou différents et peuvent représenter chacun un atome d'hydrogène, un groupe alcane(C₁-C₅)sulfonyloxy, trifluorométhanesulfonyloxy, alcane(C₁-C₅)-sulfonylamino, trifluorométhanesulfonylamino, N-alkyl(C₁-C₅)-alcane(C₁-C₅)-sulfonylamino, N-alkyl(C₁-C₅)-trifluorométhanesulfonylamino, alkyl(C₁-C₅)-sulfénylméthyle, alkyl(C₁-C₅)-sulfinylméthyle ou alkyle(C₁-C₅)-sulfonylméthyle, un groupe carbonyle substitué par un groupe hydroxy, alcoxy(C₁-C₅), amino, alkyl(C₁-C₅)amino ou dialkyl(C₁-C₅)amino, un groupe sulfonyle substitué par un groupe amino, alkyl(C₁-C₅)amino, dialkyl(C₁-C₅)amino ou iminocyclique, un groupe méthylène en position 4 pouvant être substitué par un atome de soufre ou d'oxygène, un groupe alkyl(C₁-C₅)carbonylamino, aminocarbonylamino ou alkyl(C₁-C₅)aminocarbonylamino, un groupe alkyl(C₁-C₅)mercapto, alkyl(C₁-C₅)sulfinyle ou alkyl(C₁-C₅)sulfonyle, un groupe nitro, halogéno, amino, hydroxy, alkyle(C₁-C₅), alcoxy(C₁-C₅), alcényloxy(C₁-C₅), alcynyloxy(C₁-C₅), cyanoalkyloxy(C₁-C₅), carboxyalkyl(C₁-C₅)oxy, alcoxy(C₁-C₅)-carbonyl-alkyl(C₁-C₅)oxy, dialkyl(C₁-C₅)amino, 1-imidazolyle, trifluorométhyle ou cyano, ou
R₁ représente un reste naphtyle ou un hétérocycle à cinq chaînons, saturé ou insaturé, comportant 1-4 hétéroatomes, ou un hétérocycle à six chaînons, saturé ou insaturé, comportant 1-5 hétéroatomes, les hétéroatomes pouvant être identiques ou différents et représentent l'oxygène, le soufre ou l'azote et éventuellement, peuvent porter un atome d'oxygène sur un ou plusieurs atomes d'azote, et les cycles à cinq ou six chaînons peuvent être également substitués par un ou plusieurs groupes alkyle(C₁-C₆), alcoxy(C₁-C₆), alkyl(C₁-C₆)mercapto, hydroxy, nitro, amino, halogéno ou cyano, ou peuvent être condensés avec un noyau phényle pour former un bicycle,
ou dans le cas où X représente une liaison, R₁ peut représenter, outre les groupes mentionnés ci-dessus, également un atome d'hydrogène, un groupe alkyle(C₁-C₁₀), cycloalkyle(C₃-C₇), alcényle(C₂-C₁₀), cycloalcényle(C₃-C₇), alcoxy(C₁-C₆)alkyle(C₁-c₆), carboxyalkyle(C₁-C₆), alcoxy(C₁-C₆)carbonylalkyle(C₁-C₆), aminoalkyle(C₁-C₆), hydroxyalkyle(C₁-C₆), amino, alkyl(C₁-C₆)amino, alkyl(C₁-C₆)carbonylamino, formylamino, alkyl(C₁-C₆)sulfonylamino ou un groupe alcynyle comportant à au plus 10 atomes de C,
R₂ représente un groupe alkyle(C₁-C₆) ou phényle,
R₃ représente un groupe alkyle(C₁-C₆) ou phényle ou forme, conjointement avec R₂, un groupe cycloalcényle(C₃-C₇),
R₄ représente un hétérocycle à cinq chaînons comportant 1-4 hétéroatomes ou un hétérocycle à six chaînons comportant 1-5 hétéroatomes, les hétéroatomes des cycles à cinq et six chaînons mentionnés précédemment pouvant être identiques ou différents et représentent l'azote, l'oxygène ou le soufre, et les cycles à cinq et six chaînons mentionnés ci-dessus peuvent être également substitués par un ou plusieurs groupes alkyle(C₁-C₆), alcoxy(C₁-C₆), alcoxy(C₁-C₆)alkyle(C₁-C₆), alkylmercapto(C₁-C₆), hydroxy, hydroxyalkyle(C₁-C₆), amino, halogéno, oxo ou cyano,
X représente une liaison, un groupe alcényle(C₁-C₄), le groupe vinylène, le groupe imino -NH- ou le groupe carbonylamino -CONH-,
leurs tautomères et leurs sels physiologiquement acceptables formés avec des acides organiques ou inorganiques.

2. Composés de formule I selon la revendication 1, dans lesquels
R₁ représente un noyau phényle de formule II, dans laquelle R₅ représente un atome d'hydrogène, un groupe alkyl(C₁-C₂)sulfonyloxy, trifluorométhylsulfonyloxy, alkyl(C₁-C₂)-sulfénylméthyle, alkyle(C₁-C₂)sulfynylméthyle, alkyl(C₁-C₂)-sulfonylméthyle, alkyle(C₁-C₂)sulfonylamino, N-alkyl-(C₁-C₂)-alkyl(C₁-C₂)sulfonylamino, trifluorométhylsulfonylamino ou un groupe N-alkyl(C₁-C₂)trifluorométhylsulfonylamino, un groupe carbonyle substitué par un groupe hydroxy, alcoxy(C₁-C₂), amino, alylamino(C₁-C₂) ou dialkyl(C₁-C₂)amino, ou un groupe sulfonyle substitué par un groupe amino, dialkyl(C₁-C₂)amino ou morpholino, un groupe nitro, cyano ou alkyl(C₁-C₄)aminosulonyle, un groupe alkyl(C₁-C₂)carbonylamino, aminocarbonylamino ou N-alkyl(C₁-C₂)-aminocarbonylamino, un groupe alkyl(C₁-C₂)mercapto, alkyl(C₁-C₂)-sulfinyle ou alkyl(C₁-C₂)sulfonyle, un groupe halogéno, amino, hydroxy, dialkyl(C₁-C₃)amino, alkyle(C₁-C₃), alcoxy(C₁-C₃), alcényloxy(C₂-C₃) ou alcynyloxy(C₂-C₃), un groupe cyanométhyloxy ou méthoxycarbonylméthoxy, le groupe trifluorométhyle ou le groupe 1-imidazolyle,
et
R₆ représente un atome d'hydrogène, un groupe alkyle(C₁-C₃), un groupe alcoxy(C₁-C₂) ou dialkyl(C₁-C₂)amino ou un atome d'halogène et
R₇ représente un atome d'hydrogène ou le groupe méthoxy,
ou
R₁ représente un reste pyrrole, furane, thiophène, pyrazole, imidazole, thiazole, isothiazole, oxazole, isoxazole, triazole, tétrazole, thiadiazole, oxadiazole, pyrazine, N,N-dioxypyrazine, pyrimidine, N,N-dioxy-pyrimidine, pyridazine, oxazine, thiazine, triazine, tétrazine, pyridine, N-oxy-pyridine, pipéridine, pipérazine, morpholine et thiomorpholine,
ou
dans le cas où X représente une liaison, R₁ représente, outre les groupes mentionnés ci-dessus, également un atome d'hydrogène, un groupe alkyle(C₁-C₁₀), cycloalkyle(C₃-C₇), alcényle(C₂-C₁₀), cycloalcényle(C₃-C₇), alcynyle(C₂-C₁₀), alcoxy(C₁-C₆)-alkyle(C₁-C₆), carboxyalkyle(C₁-C₆), alcoxy(C₁-C₆)-carbonylalkyle(C₁-C₆), aminoalkyle(C₁-C₆), hydroxyalkyle(C₁-C₆), amino, alkyl(C₁-C₆)amino, alkyl(C₁-C₆)carbonylamino, formylamino ou alkyl(C₁-C₆)sulfonylamino,
R₂ représente une chaîne alkyle(C₁-C₅) droite ou ramifiée,
R₃ représente une chaîne alkyle(C₁-C₅) droite ou ramifiée, ou conjointement avec R₂, une chaîne cycloalcényle(C₃-C₇),
R₄ représente un reste 3-oxy-2,3-dihydro-6-pyridazinyle, 5-alkyl(C₁-C₆)3-oxo-2,3-dihydro-6-pyridazinyle, 3-oxo-2,3,4,5-tétrahydro-6-pyridazinyle, 5-alkyl(C₁-C₆)-3-oxo-2,3,4,5-tétrahydro-6-pyridazinyle, 5-hydroxyalkyl(C₁-C₆)-3-oxo-2,3,4,5-tétrahydro-6-pyridazinyle, 3-cyano-6-alkyl(C₁-C₆)-2-oxo-1,2-dihydro-5-pyridinyle, 6-alkyl(C₁-C₆)-2-oxo-1,2-dihydro-5-pyridinyle, 3-amino-2-oxo-1,2-dihydro-5-pyridinyle, 3-amino-6-alkyl(C₁-C₆)-2-oxo-1,2-dihydro-5-pyridinyle, 3-hydroxy-6-alkyl-(C₁-C₆)-2-oxo-1,2-dihydro-5-pyridinyle, 3-oxo-2H-3,4-dihydro-1,4-thiazine-6-yle, 6-oxo-1,6-dihydro-1,2,4-triazine-3-yle, 6-oxo-1,4,5,6-tétrahydro-1,2,4-triazine-3-yle, 5-oxo-4,5-dihydro-6H-1,3,4-oxadiazine-2-yle, 5-oxo-4,5-dihydro-6H-1,3,4-thiadiazine-2-yle, 3-oxo-2,3-dihydro-1,2,4-triazine-6-yle, 3-oxo-2,3,4,5-tétrahydro-1,2,4-triazine-6-yle, 2-oxo-2,3-dihydro-6H-1,3,4-oxadiazine-5-yle, 2-oxo-2,3-dihydro-6H-1,3,4-thiadiazine-5-yle, 5-alkyl(C₁-C₆)-3-oxo-2,3,4,5-tétrahydro-1,2,4-triazine-6-yle, 2-oxo-1,2-dihydro-5-pyrimidinyle, 4-alkyl(C₁-C₆)-2-oxo-1,2-dihydro-5-pyrimidinyle, 2-oxo-1,2-dihydro-5-pyrazinyle, 3-alkyl(C₁-C₆)-2-oxo-1,2-dihydro-5-pyrazinyle, 6-alkyl(C₁-C₆)-2-oxo-1,2-dihydro-5-pyrazinyle, 4,4-dialkyl(C₁-C₆)-5-oxo-4,5-dihydro-3-pyrazolyle, 2-oxo-4-pyrrolidinyle, 3-alkyl(C₁-C₆)-2-oxo-4-pyrrolidinyle, 5-oxo-4,5-dihydro-1,2,4-triazol-3-yle, 4-alkyl(C₁-C₆)-5-oxo-4,5-dihydro-1,2,4-triazole-3-yle, 2-oxo-2,3-dihydro-4(5)-imidazolyle ou 5(4)-alkyl(C₁-C₆)-2-oxo-2,3-dihydro-4(5)-imidazolyle,
X représente une liaison, un groupe alcényle(C₁-C₂), le groupe vinylène, un groupe imino -NH- ou un groupe carbonylamino -CONH-.

3. Composés de formule générale I selon la revendication 1 ou 2, dans laquelle
R₁ représente un groupe phényle de formule générale II, dans laquelle R₅ représente un atome d'hydrogène, le groupe méthanesulfonyloxy, trifluorométhanesulfonyloxy, méthanesulfonylamino, trifluorométhanesulfonylamino, méthylmercapto, méthylsulfinyle, méthylsulfonyle, hydroxy, méthyle, méthoxy, propargyloxy, trifluorométhyle ou 1-imidazolyle, R₆ représente un atome d'hydrogène, le groupe méthyle, méthoxy, diméthylamino ou chloro et R₇ représente un atome d'hydrogène ou le groupe méthoxy, ou
R₁ représente le groupe pyrrole, furane, thiophène, pyrazole, imidazole, isothiazole, thiazole, oxazole, triazole, tétrazole, thiadiazole, isoxazole, oxadiazole, pyridine, N-oxy-pyridine, pyrazine, N,N-dioxypyrazine, pyrimidine, N,N-dioxypyrimidine, pyridazine, oxazine, thiazine, triazine, tétrazine, pipéridine, pipérazine, morpholine ou thiomorpholine, ainsi que leurs dérivés substitués par méthyle, éthyle, méthoxy, éthoxy, méthylmercapto, éthylmercapto et chloro, ou R₁ représente le reste indole, indazole, quinoléine, isoquinoléine ou naphtyle, ou
R₁ peut également représenter, dans le cas où X représente une liaison, en plus des groupes mentionnés, un atome d'hydrogène, le groupe méthyle, éthyle, propyle, butyle, pentyle, hexyle, propényle, cyclopentyle, cyclohexyle, méthylamino, amino, acétamido ou formamido,
R₂ et R₃ sont identiques et représentent un groupe méthyle, ou R₂ et R₃ forment, conjointement avec l'atome de carbone auquel ils sont liés, un cycle cylcopentane ou cyclohexane,
R₄ représente le reste 3-oxo-2,3,4,5-tétrahydro-6-pyridazinyle, 5-méthyl-3-oxo-2,3,4,5-tétrahydro-6-pyridazinyle, 5-hydroxyméthyl-3-oxo-2,3,4,5-tétrahydro-6-pyridazinyle, 3-oxo-2,3-dihydro-6-pyridazinyle, 3-cyano-6-méthyl-2-oxo-1,2-dihydro-5-pyridinyle, 3-oxo-2,3,4,5-tétrahydro-1,2,4-triazine-6-yle, 2-oxo-4,5-dihydro-6H-1,3,4-oxadiazine-5-yle, 6-oxo-1,4,5,6-tétrahydro-1,2,4-triazine-3-yle, 5-oxo-4,5-dihydro-6H-1,3,4-oxadiazine-2-yle, 2-oxo-1,2-dihydro-5-pyrimidinyle, 2-oxo-1,2-dihydro-5-pyrazinyle, 4,4-diméthyl-5-oxo-4,5-dihydro-3-pyrazolyle, 2-oxo-4-pyrrolidinyle, 4-méthyl-5-oxo-4,5-dihydro-1,2,4-triazol-3-yle ou le reste 5(4)-méthyl-2-oxo-2,3-dihydro-4(5)-imidazolyle, et
X représente une liaison, le groupe vinylène, le groupe méthylène, le groupe imino ou le groupe carbonylamino.

4. Composés de formule générale I selon la revendication 1, 2 ou 3, dans laquelle
R₁ représente un noyau phényle non substitué ou substitué par des groupes alkyle(C₁-C₅), alcoxy(C₁-C₅), halogène ou alkyl(C₁-C₅)mercapto, ou un groupe pyridyle ou thiényle, ou dans le cas où X représente une liaison ou le groupe imino -NH-, R₁ représente également un groupe alkyle(C₁-C₆),
R₂ et R₃ sont identiques et représentent un groupe méthyle,
ou
R₂ et R₃ forment, conjointement avec l'atome de carbone auquel ils sont liés, un cycle cylcopentane ou cyclohexane,
R₄ représente le reste 3-oxo-2,3,4,5-tétrahydro-6-pyridazinyle, 5-méthyl-3-oxo-2,3,4,5-tétrahydro-6-pyridazinyle ou 3-oxo-2,3-dihydro-6-pyridazinyle, et
X représente une liaison.

5. Procédé de préparation des composés de formule I selon les revendications 1-4, caractérisé en ce que, de manière connue en soi,
A) on cyclise des composés de formule générale VI 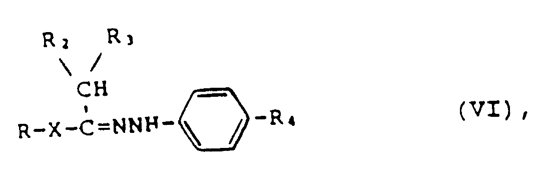 dans laquelle R₁, R₂, R₃, R₄ et X ont les significations mentionnées dans les revendications 1-4, par une synthèse Fischer-indol,
ou
B) dans le cas où R₄ représente un reste pyridazinyle éventuellement substitué une ou plusieurs fois par des groupes alkyle(C₁-C₆), alcoxy(C₁-C₆), hydroxy ou oxo, on fait réagir des composés de formule générale XI 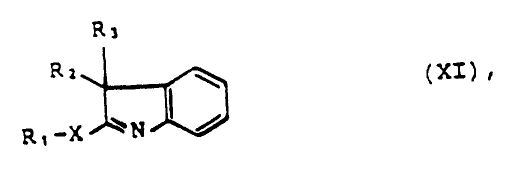 dans laquelle R₁, R₂, R₃, R₄ et X ont les significations mentionnées dans les revendications 1-4, avec des chlorures d'acide de formule générale XII
R₈OOC-CH₂-CHR₉-COCl (XII),
dans laquelle R₈ représente un groupe alkyle(C₁-C₆) et R₉ représente un atome d'hydrogène ou un groupe alkyle(C₁-C₆), ou avec des anhydrides d'acide carboxylique de formule générale XIII 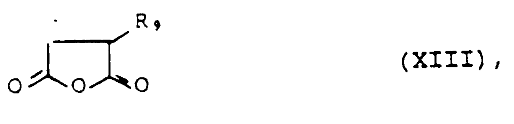 dans laquelle R₉ a la signification mentionnée ci-dessus, pour obtenir les composés de formule générale XIV ou leurs esters R₈ 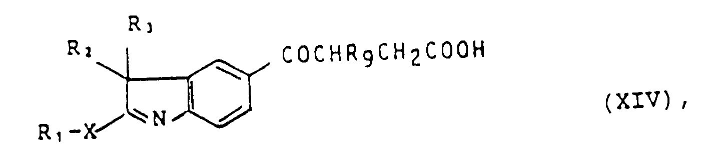 où R₁, R₂, R₃, R₉ et X ont les significations mentionnées ci-dessus, et on cyclise les composés ainsi obtenus avec de l'hydrazine ou avec un dérivé approprié de l'hydrazine, pour obtenir des composés de formule générale I, dans laquelle R₄ représente un reste pyridazinyle éventuellement substitué par un groupe alkyle(C₁-C₆), alcoxy(C₁-C₆), hydroxy ou oxo,
et ensuite, on transforme éventuellement les composés ainsi obtenus, de formule générale I, en d'autres composés de formule générale I, et l'on transforme ces composés en leurs sels pharmacologiquement acceptables.

6. Médicaments contenant au moins un composé de formule I selon l'une quelconque des revendications 1-4, ainsi qu'éventuellement, des véhicules et adjuvants usuels pharmacologiquement acceptables.

7. Utilisation des composés de formule I selon l'une quelconque des revendications 1-4, pour la préparation de médicaments ayant une action inotrope positive.

8. Procédé de préparation de médicaments contenant au moins un composé de formule I selon l'une quelconque des revendications 1-4, caractérisé en ce qu'on mélange un composé de formule I avec des véhicules et adjuvants usuels pharmacologiquement acceptables.

9. Composés de formule VI 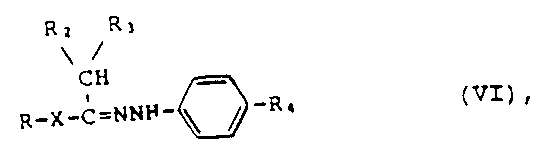 dans laquelle
R₁ représente un noyau phényle de formule générale II 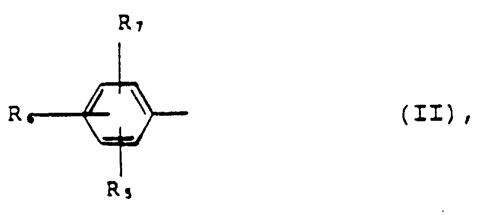 dans laquelle
R₅, R₆, R₇ peuvent être identiques ou différents et peuvent représenter chacun un atome d'hydrogène, un groupe alcane(C₁-C₅)sulfonyloxy, trifluorométhanesulfonyloxy, alcane(C₁-C₅)-sulfonylamino, trifluorométhanesulfonylamino, N-alkyl(C₁-C₅)-alcane(C₁-C₅)-sulfonylamino, N-alkyl(C₁-C₅)-trifluorométhanesulfonylamino, alkyl(C₁-C₅)-sulfénylméthyle, alkyl(C₁-C₅)-sulfinylméthyle ou alkyle(C₁-C₅)-sulfonylméthyle, un groupe carbonyle substitué par un groupe hydroxy, alcoxy(C₁-C₅), amino, alkyl(C₁-C₅)amino ou dialkyl(C₁-C₅)amino, un groupe sulfonyle substitué par un groupe amino, alkyl(C₁-C₅)amino, dialkyl(C₁-C₅)amino ou iminocyclique, un groupe méthylène en position 4 pouvant être substitué par un atome de soufre ou d'oxygène, un groupe alkyl(C₁-C₅)carbonylamino, aminocarbonylamino ou alkyl(C₁-C₅)aminocarbonylamino, un groupe alkyl(C₁-C₅)mercapto, alkyl(C₁-C₅)sulfinyle ou alkyl(C₁-C₅)sulfonyle, un groupe nitro, halogéno, amino, hydroxy, alkyle(C₁-C₅), alcoxy(C₁-C₅), alcényloxy(C₁-C₅), alcynyloxy(C₁-C₅), cyanoalkyloxy(C₁-C₅), carboxyalkyl(C₁-C₅)oxy, alcoxy(C₁-C₅)-carbonyl-alkyl(C₁-C₅)oxy, dialkyl(C₁-C₅)amino, 1-imidazolyle, trifluorométhyle ou cyano, ou
R₁ représente un reste naphtyle ou un hétérocycle à cinq chaînons, saturé ou insaturé, comportant 1-4 hétéroatomes, ou un hétérocycle à six chaînons, saturé ou insaturé, comportant 1-5 hétéroatomes, les hétéroatomes pouvant être identiques ou différents et representent l'oxygène, le soufre ou l'azote et éventuellement, peuvent porter un atome d'oxygène sur un ou plusieurs atomes d'azote, et les cycles à cinq ou six chaînons peuvent être également substitués par un ou plusieurs groupes alkyle(C₁-C₆), alcoxy(C₁-C₆), alkyl(C₁-C₆)mercapto, hydroxy, nitro, amino, halogéno ou cyano, ou peuvent être condensés avec un noyau phényle pour former un bicycle,
ou dans le cas où X représente une liaison, R₁ peut représenter, outre les groupes mentionnés ci-dessus, également un atome d'hydrogène, un groupe alkyle(C₁-C₁₀), cycloalkyle(C₃-C₇), alcényle(C₂-C₁₀), cycloalcényle(C₃-C₇), alcoxy(C₁-C₆)alkyle(C₁-C₆), carboxyalkyle(C₁-C₆), alcoxy(C₁-C₆)carbonylalxyle(C₁-C₆), aminoalkyle(C₁-C₆), hydroxyalkyle(C₁-C₆), amino, alkyl(C₁-C₆)amino, alkyl(C₁-C₆)carbonylamino, formylamino, alkyl(C₁-C₆)sulfonylamino ou un groupe alcynyle comportant à au plus 10 atomes de C,
R₂ représente un groupe alkyle(C₁-C₆) ou phényle,
R₃ représente un groupe alkyle(C₁-C₆) ou phényle ou forme, conjointement avec R₂, un groupe cycloalcényle(C₃-C₇),
R₄ représente un hétérocycle à cinq chaînons comportant 1-4 hétéroatomes ou un hétérocycle à six chaînons comportant 1-5 hétéroatomes, les hétéroatomes des cycles à cinq et six chaînons mentionnés précédemment pouvant être identiques ou différents et représentent l'azote, l'oxygène ou le soufre, et les cycles à cinq et six chaînons mentionnés ci-dessus peuvent être également substitués par un ou plusieurs groupes alkyle(C₁-C₆), alcoxy(C₁-C₆), alcoxy(C₁-C₆)alkyle(C₁-C₆), alkylmercapto(C₁-C₆), hydroxy, hydroxyalkyle(C₁-C₆), amino, halogéno, oxo ou cyano,
X représente une liaison, un groupe alcényle(C₁-C₄), le groupe vinylène, le groupe imino -NH- ou le groupe carbonylamino -CONH-,
à l'exception du composé 6-(4-sec.-butylidène-hydrazinophényl)-5-méthyl-4,5-dihydro-1,2,4-triazine-3(2H)-one.
